# EUROPEAN PATENT APPLICATION

(11) **EP 1 500 648 A1**
(43) Date of publication of application: **26.01.2005**
(21) Application number: 03725627.8
(22) Date of filing: 22.04.2003
(51) Int. Cl.: C07D 211/26, C07D 401/04, C07D 401/12, A61K 31/451, A61K 31/4545, A61K 31/454, A61P 3/06, A61P 9/10, A61P 43/00

(54) **NOVEL PIPERIDINE DERIVATIVE**

(30) Priority: 25.04.2002 JP 2002124311
(71) Applicant: Sumitomo Pharmaceuticals Company, Limited, Osaka-shi, Osaka 541-8510 (JP)
(72) Inventor: BAN, Hitoshi, Nishinomiya-shi, Hyogo 662-0837 (JP); MURAOKA, Masami, Suita-shi, Osaka 565-0811 (JP)
(74) Representative: VOSSIUS & PARTNER
(86) International application number: PCT/JP2003/005124
(87) International publication number: WO 2003/091216

(57) **Abstract**

A compound of the formula (1): 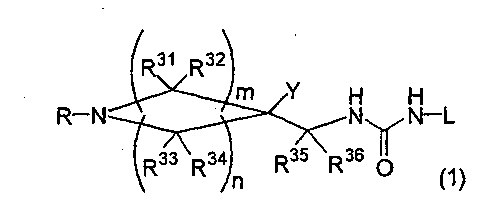 wherein m and n are independently an integer of 0 to 4, and m+n=4; L is cycloalkyl, substituted cycloalkyl, aromatic group, or substituted aromatic group; Y is aryl or substituted aryl; R is hydrogen, alkyl, etc.; R³¹, R³², R³³, R³⁴, R³⁵ and R³⁶ are the same or different and are selected, if two or more exist, independently from hydrogen, alkyl, etc.,
or a prodrug thereof, or a pharmaceutically acceptable salt of the same, which exhibits ACAT inhibitory activity, and is useful as an agent for treatment of hyperlipidemia and atherosclerosis.

## Description

### TECHNICAL FIELD

The present invention relates to a compound, a prodrug thereof, or a pharmaceutically acceptable salt of the same, which exhibits an acyl-CoA: cholesterol acyl transferase (ACAT) inhibitory activity, and is useful as an agent for treatment of hyperlipidemia and atherosclerosis, and a use thereof.

### BACKGROUN ART

Cerebrovascular disorders such as stroke and myocardial infarction, which rank in high in causes of death in developed countries, break out with being accompanied by atherosclerosis as a basal disease. In accordance with epidemiology research, it is pointed out that hypercholesterolemia is one of risk factors for atherosclerosis, and there are mainly used anti-hyperlipidemic agents, which can reduce cholesterol level in blood, in the prophylaxis or treatment thereof. However, there is no sufficiently effective agent in terms of the efficacy thereof. Recently, it is found that cells derived from macrophage accumulate cholesterol ester droplet within the cells and become foam cells in atherosclerotic lesions, and it is clarified that these foam cells deeply participate in the developments of atherosclerotic lesions (Arteriosclerosis, 10, 164-177, 1990). In addition, it is reported that ACAT activity is increased in the vascular wall of atherosclerotic lesions and thereby cholesterol esters are accumulated in the vascular wall (Biochem. Biophys. Acta, 617, 458-471, 1980). Therefore, an inhibitor of ACAT, which catalyzes cholesterol esterification, is expected to suppress the formation or the development of atherosclerotic lesions as a result of the inhibition of foam cell formation and of cholesterol ester accumulation in lesions.

On the other hand, cholesterol in food is absorbed in the free form at intestinal epidermal cells, esterified by ACAT and then released in the form of chylomicron into the blood. Therefore, an inhibitor of ACAT is expected to reduce the cholesterol level in the blood by the inhibition of absorption of cholesterol in food at the intestine and also inhibition of the reabsorption of cholesterol released into the intestine (J. Lipid. Research, 34, 279-294, 1993).

JP-A-2-6456, JP-A-2-6457 and EP 0293880 disclose phenylurea derivatives having an ACAT inhibitory activity, but any of these derivatives are structurally different in the chemical structure from the compound of the present invention.

### DISCLOSURE OF INVENTION

An object of the present invention is to provide a compound having an ACAT inhibitory activity and being useful as an agent for treatment of hyperlipidemia and atherosclerosis.

The present inventors have intensively studied in order to solve the above-mentioned problems, and have found that a piperidine derivative of the following formula (1), a prodrug thereof, or a pharmaceutically acceptable salt of the same exhibits a potent ACAT inhibitory activity, and they have accomplished the present invention. The compound of the present invention is a novel compound having a different structure from the known compounds as disclosed above.

That is, the present invention relates to the following embodiments:
[1] A compound of the formula (1): 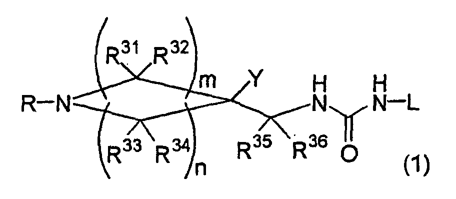 wherein m and n are independently an integer of 0 to 4, and m+n=4,
   L is a cycloalkyl group, a substituted cycloalkyl group, an aromatic group, or a substituted aromatic group,
   Y is an aryl group or a substituted aryl group,
   R is a hydrogen atom, an alkyl group, a substituted alkyl group, an alkenyl group, a substituted alkenyl group, an alkynyl group, a substituted alkynyl group, a cycloalkyl group, a substituted cycloalkyl group, an aromatic group, a substituted aromatic group, or a group of the formula: -C(=O)R² (R² is an alkyl group, a substituted alkyl group, an alkenyl group, a substituted alkenyl group, an alkynyl group, a substituted alkynyl group, a cycloalkyl group, a substituted cycloalkyl group, an aromatic group, or a substituted aromatic group),
   R³¹, R³², R³³ and R³⁴ are the same or different, and are selected, if two or more thereof exist, independently from a hydrogen atom, an alkyl group, a substituted alkyl group, a hydroxy group, an alkoxy group, and an aralkyloxy group, or a combination of R³¹ and R³², and/or a combination of R³³ and R³⁴ may combine each other and form an oxo group,
   R³⁵ and R³⁶ are the same or different, and are selected, if both exist, independently from a hydrogen atom, an alkyl group and a substituted alkyl group, or R³⁵ and R³⁶ may combined each other and form an oxo group,
   or a prodrug thereof, or a pharmaceutically acceptable salt of the same.
[2] The compound according to the above [1], or a prodrug thereof, or a pharmaceutically acceptable salt of the same, wherein Y is a phenyl group or a substituted phenyl group.
[3] The compound according to the above [2], or a prodrug thereof, or a pharmaceutically acceptable salt of the same, wherein L is a substituted phenyl group.
[4] The compound according to the above [3], or a prodrug thereof, or a pharmaceutically acceptable salt of the same, wherein L is a group of the formula (2): 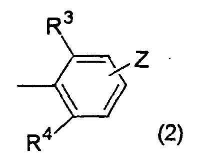 wherein R³ and R⁴ are independently a substituted or unsubstituted lower alkyl group,
   Z is a hydrogen atom, a hydroxy group, a lower alkylsulfonamido group, a lower alkoxycarbonylamino group, an amino group, a lower alkylamino group, or a di-lower alkyl amino group.
[5] The compound according to the above [4], or a prodrug thereof, or a pharmaceutically acceptable salt of the same, wherein R is an aromatic group or a substituted aromatic group.
[6] The compound according to the above [5], or a prodrug thereof, or a pharmaceutically acceptable salt of the same, wherein R is a substituted phenyl group.
[7] The compound according to the above [6], or a prodrug thereof, or a pharmaceutically acceptable salt of the same, wherein Y is a substituted phenyl group or a substituted pyridyl group, and said phenyl group or pyridyl group may be substituted by one or more groups, which are the same or different and selected from a hydroxy group and a group of the formula: -O-E-A {O is an oxygen atom, E is a divalent C₁₋₈ hydrocarbon group optionally having an unsaturated bond, and A is a hydrogen atom, a hydroxy group, a carboxyl group, a lower alkoxycarbonyl group, a benzyloxycarbonyl group, a halogen atom, a cyano group, a trifluoromethyl group, an aralkyloxy group, an aryloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, or a group of the formula:-NR⁶R⁷ (R⁶ and R⁷ are independently a hydrogen atom, a lower alkyl group, a lower alkoxy-substituted lower alkyl group, a cycloalkyl group, a lower alkoxycarbonyl group, a heteroarylmethyl group, or an aralkyl group, or R⁶ and R⁷ may combine each other, and with the adjacent nitrogen atom to which they bond, form a saturated cyclic amino group having 3 to 8 carbon atoms as ones forming the said ring, and optionally having one -NR⁸- (R⁸ is a hydrogen atom, a lower alkyl group, a phenyl group, a lower alkoxycarbonyl group, or a benzyl group) or one oxygen atom in the cycle thereof), a group of the formula: -C(=O)NR⁶R⁷ (R⁶ and R⁷ are as defined above), or a group of the formula:-NHC(=O)R⁹ (R⁹ is an alkyl group, a substituted alkyl group, a cycloalkyl group, or a substituted cycloalkyl group)}.
[8] The compound according to the above [7], or a prodrug thereof, or a pharmaceutically acceptable salt of the same, wherein in at least one of the substituents represented by the formula: -O-E-A on Y, E is a C₁₋₄ alkylene group.
[9] The compound according to the above [8], or a prodrug thereof, or a pharmaceutically acceptable salt of the same, wherein in at least one of the substituents represented by the formula: -O-E-A on Y, A is a hydrogen atom or a hydroxy group.
[10] The compound according to the above [9], or a prodrug thereof, or a pharmaceutically acceptable salt of the same, wherein Z is a hydrogen atom or an amino group.
[11] The compound according to the above [10], or a prodrug thereof, or a pharmaceutically acceptable salt of the same, wherein R³ and R⁴ are independently an unsubstituted lower alkyl group.
[12] The compound according to the above [11], or a prodrug thereof, or a pharmaceutically acceptable salt of the same, wherein R is a substituted phenyl group or a substituted pyridyl group, and said phenyl group or pyridyl group may be substituted by one or more groups, which are the same or different and selected from a hydroxy group and a group of the formula: -O-E-A {O is an oxygen atom, E is a divalent C₁₋₈ hydrocarbon group optionally having an unsaturated bond, and A is a hydrogen atom, a hydroxy group, a carboxyl group, a lower alkoxycarbonyl group, a benzyloxycarbonyl group, a halogen atom, a cyano group, a trifluoromethyl group, an aralkyloxy group, an aryloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, or a group of the formula: -NR⁶R⁷ (R⁶ and R⁷ are independently a hydrogen atom, a lower alkyl group, a lower alkoxy-substituted lower alkyl group, a cycloalkyl group, a lower alkoxycarbonyl group, a heteroarylmethyl group, or an aralkyl group, or R⁶ and R⁷ may combine each other, and with the adjacent nitrogen atom to which they bond, form a saturated cyclic amino group having 3 to 8 carbon atoms as ones forming the said ring, and optionally having one -NR⁸- (R⁸ is a hydrogen atom, a lower alkyl group, a phenyl group, a lower alkoxycarbonyl group, or a benzyl group) or one oxygen atom in the cycle thereof), or a group of the formula: -C(=O)NR⁶R⁷ (R⁶ and R⁷ are as defined above)}.
[ 13] The compound according to the above [12], or a prodrug thereof, or a pharmaceutically acceptable salt of the same, wherein in at least one of the substituents represented by the formula:-O-E-A on R, E is a C₁₋₄ alkylene group.
[14] The compound according to the above [13], or a prodrug thereof, or a pharmaceutically acceptable salt of the same, wherein in at least one of the substituents represented by the formula:-O-E-A on R, A is a hydrogen atom, a hydroxy group, a lower alkoxy group, a lower alkanoyloxy group, a lower alkanoylamino group, a lower alkoxycarbonylamino group, or a group of the formula: -NR⁶R⁷ or the formula: -C(=O)NR⁶R⁷.
[15] The compound according to the above [14], or a prodrug thereof, or a pharmaceutically acceptable salt of the same, wherein in at least one of the substituents represented by the formula:-O-E-A on R, A is a hydrogen atom, a hydroxy group, a lower alkoxy group, or a group of the formula: -NR⁶R⁷ (R⁶ and R⁷ are independently a hydrogen atom or a lower alkyl group).
[16] The compound according to the above [15], or a prodrug thereof, or a pharmaceutically acceptable salt of the same, wherein R³ and R⁴ are an isopropyl group.
[17] The compound according to any one of the above [1] to [16], or a prodrug thereof, or a pharmaceutically acceptable salt of the same, wherein m is 2, n is 2, and all of R³¹, R³², R³³, R³⁴, R³⁵ and R³⁶ are a hydrogen atom.
[18] A pharmaceutical composition comprising as an active ingredient the compound as set forth in any one of the above [1] to [17], or a prodrug thereof, or a pharmaceutically acceptable salt of the same.
[18] An acyl-CoA: cholesterol acyl transferase (ACAT) inhibitor, which comprises as an active ingredient the compound as set forth in any one of the above [1] to [17], or a prodrug thereof, or a pharmaceutically acceptable salt of the same.
[20] An agent for treatment of hyperlipidemia or atherosclerosis, which comprises as an active ingredient the compound as set forth in any one of the above [1] to [17], or a prodrug thereof, or a pharmaceutically acceptable salt of the same.
[21] A method for treatment of hyperlipidemia or atherosclerosis in a patient in need, which comprises administering a therapeutically effective amount of the compound as set forth in any one of the above [1] to [17], or a prodrug thereof, or a pharmaceutically acceptable salt of the same, to said patient.
[22] A use of the compound as set forth in any one of the above [1] to [ 17], or a prodrug thereof, or a pharmaceutically acceptable salt of the same, in preparation of an agent for treatment of hyperlipidemia or atherosclerosis.

### BEST MODE FOR CARRYING OUT THE INVENTION

Each group in the present invention is explained below. Unless defined otherwise, the definition for each group should be applied to cases wherein said group is a part of another substituent.

The alkyl group or the alkyl moiety of a substituted alkyl group may include, for example, a straight chain or branched chain C₁₋₁₅ alkyl group, and examples thereof are methyl, ethyl, propyl, 2-propyl, butyl, 2-butyl, 2-methylpropyl, 1,1-dimethylethyl, pentyl, 3-pentyl, 3-methylbutyl, hexyl, 3-hexyl, 4-methylpentyl, 4-heptyl, octyl, 4-octyl, decyl, etc., and preferable one is a straight chain or branched chain C₁₋₆ alkyl group.

The alkenyl group or the alkenyl moiety of a substituted alkenyl group may include, for example, a straight chain or branched chain C₂₋₁₅ alkenyl group, and examples thereof are vinyl, allyl, 2-propenyl, 2-methyl-2-propenyl, 2-butenyl, 3-butenyl, 3-methyl-2-butenyl, 4-pentenyl, 3-hexenyl, 3-ethyl-2-pentenyl, 4-ethyl-3-hexenyl, etc.

The alkynyl group or the alkynyl moiety of a substituted alkynyl group may include, for example, a straight chain or branched chain C₃₋₁₅ alkynyl group, and examples thereof are 2-propinyl, 3-butynyl, 4-pentynyl, 3-hexynyl, 5-methyl-2-hexynyl, 6-methyl-4-heptynyl, etc.

The substituted alkyl group, the substituted alkenyl group, and the substituted alkynyl group may have one or more substituents which are the same or different, and the substituents are, for example, a substituted or unsubstituted aromatic group, a substituted or unsubstituted cycloalkyl group, a halogen atom, a cyano group, a hydroxy group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkylsulfonamido group, etc.

The halogen atom is, for example, fluorine atom, chlorine atom, bromine atom, or iodine atom, and preferable halogen atom is fluorine atom.

The cycloalkyl group or the cycloalkyl moiety of a substituted cycloalkyl group includes, for example, a C₃₋₈ cycloalkyl group, and examples thereof are cyclopropyl, cyclobutyl, cyclopentyl, cyclohexyl, cycloheptyl, cyclooctyl, etc.

The substituted cycloalkyl group may have one or more substituents which are the same or different, and the substituents are, for example, a lower alkyl group, a halogen atom, a cyano group, a hydroxy group, a lower alkoxy group, a lower alkanoyloxy group, an amino group, a mono-lower alkylamino group, a di-lower alkylamino group, a carbamoyl group, a lower alkylaminocarbonyl group, a di-lower alkylaminocarbonyl group, a lower alkoxycarbonylamino group, a carboxyl group, a lower alkoxycarbonyl group, a lower alkylthio group, a lower alkylsulfinyl group, a lower alkylsulfonyl group, a lower alkanoylamino group, a lower alkylsulfonamido group, etc. Preferable halogen atom is fluorine atom.

The term "lower" in the present invention means that an alkyl moiety described with "lower" is a lower alkyl group, and such a lower alkyl group includes a lower alkyl group having 1 to 6 carbon atoms such as methyl, ethyl, propyl, 2-propyl, butyl, pentyl, hexyl, etc. The substituent of the substituted lower alkyl moiety includes, for example, a halogen atom, a cyano group, a lower alkoxy group, etc. Preferable halogen atom is fluorine atom.

The aromatic group includes an aryl group and a heteroaryl group.

The aryl group includes, for example, an aryl group having carbon atoms of not more than 10, such as a phenyl group, a naphthyl group, etc.

The heteroaryl group includes, for example, a 5- to 6-membered monocyclic group having 1 to 2 nitrogen atoms, a 5- to 6-membered monocyclic group having 1 to 2 nitrogen atoms and one oxygen atom or one sulfur atom, a 5-membered monocyclic group having one oxygen atom or one sulfur atom, a bicyclic group formed by condensing a 6-membered ring and a 5- or 6-membered ring and having 1 to 4 nitrogen atoms, and examples thereof are 2-pyridyl, 3-pyridyl, 4-pyridyl, 2-thienyl, 3-thienyl, 3-oxodiazolyl, 1-imidazolyl, 2-imidazolyl, 2-thiazolyl, 3-isothiazolyl, 2-oxazolyl, 3-isoxazolyl, 2-furyl, 3-furyl, 3-pyrrolyl, 2-quinolyl, 8-quinolyl, 2-quinazolinyl, 8-purinyl, etc.

The substituted aromatic group may have one or more substituents, which are the same or different and selected from a halogen atom, a cyano group, a trifluoromethyl group, a nitro group, a hydroxy group, a methylenedioxy group, a lower alkyl group, a lower alkoxy group, a lower alkoxy group being substituted by a hydroxy group, a lower alkoxy group being substituted by one or more halogen atoms, a benzyloxy group, a lower alkanoyloxy group, an amino group, a mono-lower alkylamino group, a di-lower alkylamino group, a lower alkoxycarbonylamino group, a carbamoyl group, a lower alkylaminocarbonyl group, a di-lower alkylaminocarbonyl group, a carboxyl group, a lower alkoxycarbonyl group, a lower alkylthio group, a lower alkylsulfinyl group, a lower alkylsulfonyl group, a lower alkanoylamino group, a lower alkylsulfonamido group, a group of the formula: -O-E-A {O is an oxygen atom, E is a divalent C₁₋₈ hydrocarbon group optionally having an unsaturated bond, and A is a hydrogen atom, a hydroxy group, a carboxyl group, a lower alkoxycarbonyl group, a benzyloxycarbonyl group, a halogen atom, a cyano group, a benzyloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, or a group of the formula:-NR⁶R⁷ (R⁶ and R⁷ are independently a hydrogen atom, a lower alkyl group, a lower alkoxy-substituted lower alkyl group, a cycloalkyl group, a lower alkoxycarbonyl group, a heteroarylmethyl group, or an aralkyl group, or R⁶ and R⁷ may combine each other, and with the adjacent nitrogen atom to which they bond, form a saturated cyclic amino group having 3 to 8 carbon atoms as ones forming the said ring, and optionally having one -NR⁸- (R⁸ is a hydrogen atom, a lower alkyl group, a phenyl group, a lower alkoxycarbonyl group, or a benzyl group) or one oxygen atom in the cycle thereof), or a group of the formula: -C(=O)NR⁶R⁷ (R⁶ and R⁷ are as defined above)}.

The divalent C₁₋₈ hydrocarbon group optionally having an unsaturated bond includes, for example, an alkylene group (e.g., methylene, ethylene, trimethylene, tetramethylene, pentamethylene, hexamethylene, etc.), an alkenylene group (e.g., propenylene, butenylene, etc.), and an alkynylene (e.g., ethynylene, propynylene, butynylene, etc.). These groups may be either a straight chain one or a branched chain one.

The aralkyl group includes an alkyl group being substituted by the above-mentioned aryl group.

The saturated cyclic amino group having 3 to 8 carbon atoms as ones forming the said ring, and optionally having one -NR⁸- or one oxygen atom in the cycle thereof, which is formed by combining R⁶ and R⁷ with the adjacent nitrogen atom to which they bond includes, for example, 1-pyrrolidinyl, 1-piperidino, 1-piperazinyl, morpholino, 1-(4-methyl)piperazinyl, etc.

The acyl group includes, for example, a formyl group, a C₂₋₆ alkanoyl group (e.g., acetyl, propanoyl, etc.), a C₄₋₇ cycloalkanecarbonyl group (e.g., cyclopropanecarbonyl, cyclobutanecarbonyl, cyclopentanecarbonyl, cyclohexanecarbonyl, etc.), a C₃₋₆ cycloalkenecarbonyl group (e.g., cyclopentenecarbonyl, cyclohexenecarbonyl, etc.), a C₆₋₁₀ aroyl group (e.g., benzoyl, toluoyl, naphthoyl, etc.), a saturated heterocyclic-carbonyl group having a 5- or 6-membered saturated heterocyclic group containing 1 to 2 heteroatoms selected from a nitrogen atom, an oxygen atom and a sulfur atom (e.g., 2-piperidinecarbonyl, 3-morpholinecarbonyl, etc.), a heteroaromatic acyl group having a 5- or 6-membered heteroaromatic cycle containing 1 to 2 heteroatoms selected from a nitrogen atom, an oxygen atom or a sulfur atom (e.g., furoyl, thenoyl, nicotinoyl, isonicotinoyl, etc.).

The preferable group for Y is, for example, a substituted phenyl group, and more preferable group for Y is a lower alkoxy-substituted phenyl group.

The preferable group for R is, for example, a substituted or unsubstituted phenyl group, a benzyl group, a pyridyl group, 2,2,2,-trifluoroethyl group. More preferable group for R is a substituted phenyl group.

The preferable group for L is a group of the formula (2):

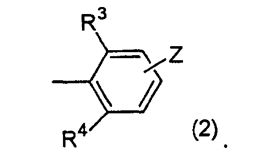

The preferable groups for R³ and R⁴ are an unsubstituted lower alkyl group, and more preferable group is an isopropyl group.

The preferable group for Z includes, for example, a hydrogen atom, a hydroxy group, and an amino group, and more preferable group for Z is an amino group.

The "prodrug" includes a compound which can easily be hydrolyzed in the living body, and can reproduce the compound of the formula (1). The "prodrug" includes, for example, when such a compound of the formula (1) has a carboxyl group, then ones wherein said carboxyl group is replaced by an alkoxycarbonyl group, an alkylthiocarbonyl group, or an alkylaminocarbonyl group, or when a compound of the formula (1) has an amino group, then ones wherein said amino group is substituted by an alkanoyl group to form an alkanoylamino group, or substituted by an alkoxycarbonyl group to form an alkoxycarbonylamino group, or converted into an acyloxymethylamino group or a hydroxyamine. When a compound of the formula (1) has a hydroxy group, the prodrug thereof is, for example, compounds wherein said hydroxy group is substituted by an acyl group as mentioned above and converted to an acyloxy group, or converted into a phosphate ester, or converted to an acyloxymethyloxy group. The alkyl moiety of groups being used for making a prodrug may be the above-mentioned alkyl groups, and said alkyl group may optionally be substituted, for example, by an alkoxy group having 1 to 6 carbon atoms, etc. Preferable examples are, to take the compounds wherein a carboxyl group is converted into an alkoxycarbonyl group as an example, a lower (e.g., having 1 to 6 carbon atoms) alkoxycarbonyl group being substituted by an alkoxy group such as methoxycarbonyl, methoxymethoxycarbonyl, ethoxymethoxycarbonyl, 2-methoxyethoxycarbonyl, 2-methoxyethoxymethoxycarbonyl, or pivaloyloxymethoxycarbonyl.

The pharmaceutically acceptable salt includes an acid addition salt, and the acid for forming an acid addition salt includes, for example, inorganic acids such as hydrochloric acid, hydrobromic acid, hydroiodic acid, nitric acid, sulfuric acid, etc., or organic acids such as formic acid, acetic acid, oxalic acid, citric acid, malic acid, tartaric acid, fumaric acid, maleic acid, methanesulfonic acid, benzenesulfonic acid, etc.

The present compounds may have a stereoisomer due to an asymmetric carbon atom thereof. In such cases, the present compounds also include each isomer or a mixture thereof.

The present compounds as mentioned above may be in the form of a free compound, a salt or a hydrate.

The compound of the above formula (1), a prodrug thereof, or a pharmaceutically acceptable salt of the same may be administered either parenterally or orally. The present compounds can be formulated into liquid preparations such as solutions, emulsions, suspensions, etc., and can be administered in the form of an injection, and if necessary, buffering agents, solubilizers, isotonic agents, etc. may be added thereto. Further, the present compounds can also be administered rectally in the form of a suppository. The present compounds can also be administered orally in the form of a conventional administration form such as tablets (sugar-coated tablets, film-coating tablets), powders, granules, capsules (including soft capsules), syrups, and suspensions. These pharmaceutical preparations can be formulated in a conventional manner by mixing an active ingredient with conventional carriers or diluents, exipients, binding agents, lubricants, disintegration agents, or stabilizers in a conventional manner. If necessary, other additives such as preservatives, antioxidant substances, coloring agents, sweetening agents may be added thereof. The dosage and the frequency of administration of the present compounds may vary according to the conditions, ages, weights of the patients and the administration form, etc., but when administered in the form of an injection, the present compounds can usually be administered in a dose of 0.1 to 100 mg per day in adult, once a day, or divided into 2 - 4 dosage units. When administered orally, the dosage is in the range of 0.1 to 1000 mg (preferablely 1 to 400 mg) once a day or divided into several dosage units (e.g., 2 to 4 times)

The compound of the present invention may be prepared by the following Processes. In each Process as mentioned below, even in cases where the use of the protecting group is not specified, when the starting compounds have a reactive functional group such as amino group as a substituent, then if necessary, the reaction may advantageously proceed by protecting these groups and removing the protecting groups after the completion of the reaction or after a series of reactions may be finished. The protection or deprotection can be carried out by the methods disclosed in the literature: PROTECTIVE GROUPS IN ORGANIC SYNTHESIS, 3rd ed., JOHN WILEY & SONS, INC.; New York (1999).

(A) A compound of the formula (8), which is a piperidinecarbonitrile having the 1-position substituted by an alkyl group or etc. represented by R¹² may be prepared as follows. In the following process, R¹² is an alkyl group, a substituted alkyl group, an alkenyl group, a substituted alkenyl group, an alkynyl group, a substituted alkynyl group, a cycloalkyl group, a substituted cycloalkyl group, an aromatic group, or a substituted aromatic group.

The substituent of the substituted alkyl group, the substituted alkenyl group, and the substituted alkynyl group may be, for example, a substituted or unsubstituted aromatic group, a substituted or unsubstituted cycloalkyl group, a fluorine atom, a trifluoromethyl group, a lower alkoxy group, a di-lower alkylamino group, etc. The substituent of the substituted cycloalkyl group may be, for example, a lower alkyl group, a fluorine atom, a trifluoromethyl group, a lower alkoxy group, a di-lower alkylamino group, etc. The substituent of the substituted aromatic group may be, for example, a fluorine atom, a trifluoromethyl group, a methylenedioxy group, a lower alkyl group, a lower alkoxy group, a lower alkoxy group being substituted by one or more fluorine atoms, a benzyloxy group, a di-lower alkylamino group, etc. 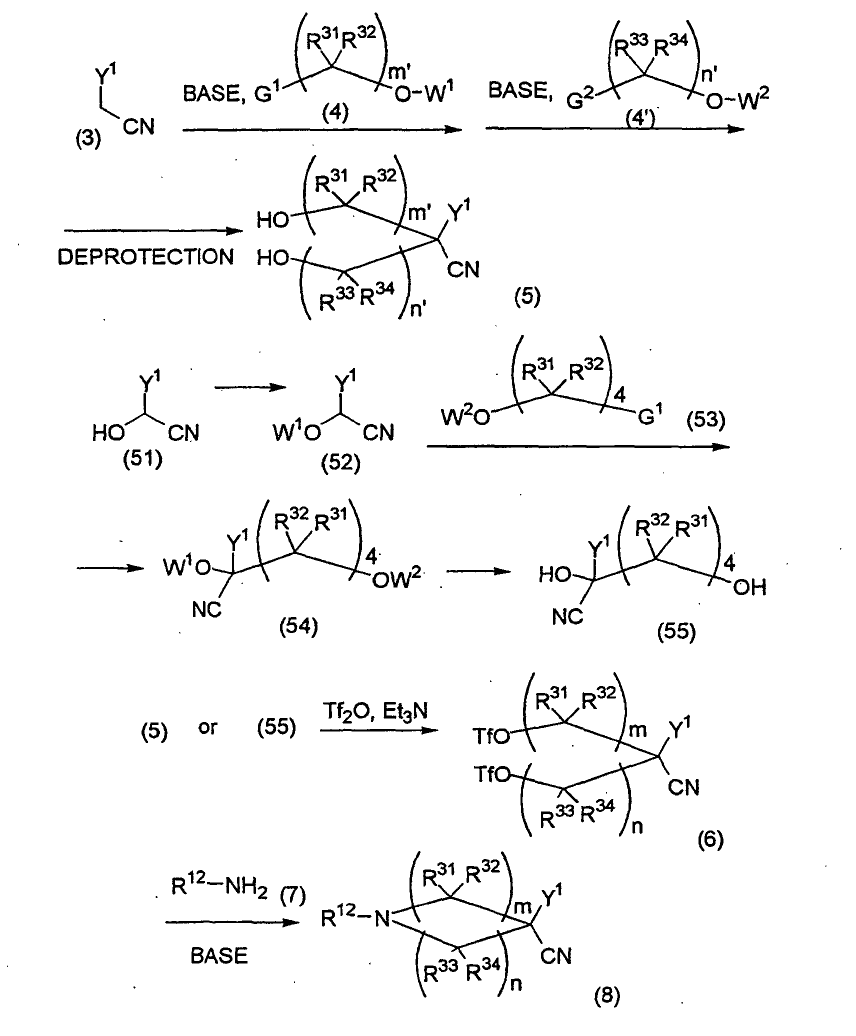 (wherein Y¹ is an aryl group or a substituted aryl group; the substituent may be a fluorine atom, a trifluoromethyl group, a methylenedioxy group, a lower alkyl group, a lower alkoxy group, a lower alkoxy group being substituted by one or more fluorine atoms, a benzyloxy group, a di-lower alkylamino group, etc.; G¹ and G² are a leaving group; W¹ and W² are a protecting group for a hydroxy group; Tf is a trifluoromethanesulfonyl group; m' and n' are independently an integer of 1 to 3, and m'+n'=4; m, n, R³¹, R³², R³³, R³⁴, and R¹² are as defined above)

For the preparation of the compound wherein the 3-position or the 4-position of the piperidine nucleus is substituted by a cyano group or Y¹, the route for preparing the compound of the formula (5) is employed. For example, the compound of the formula (3) is reacted with the compound of the formula (4) in an amount of 1 to 1.5 mole equivalent in the presence of a base such as sodium hydride in an amount of 1 to 1.5 mole equivalent or more at a temperature of from room temperature to a boiling point of the solvent to be used, and further reacted with the compound of the formula (4') in the the presence of a base such as sodium hydride in an amount of 1 to 1.5 mole equivalent or more at a temperature of from room temperature to a boiling point of the solvent to be used, and the protecting group for a hydroxy group of the resultant is removed to give the compound of the formula (5). In the cases of m'=n'=2, the compound of the formula (4) may be reacted with the compound of the formula (4) in an amount of 2 to 3 mole equivalents in the presence of a base such as sodium hydride in an amount of 2 to 3 mole equivalents.

The above reaction is usually carried out in a solvent, and the solvent may be any one which does not disturb the reaction, for example, ethers (e.g., ethyl ether, dimethoxyethane, isopropyl ether, tetrahydrofuran, dioxane, etc.), and dimethylsulfoxide.

On the other hand, for the preparation of the compound wherein the 2-position of the piperidine nucleus is substituted by a cyano group and Y¹, the route for preparing the compound of the formula (55) is employed. For example, the hydroxy group of the compound of the formula (51) is protected to give the compound (52), which is reacted with the compound of the formula (53) in an amount of 1 to 15 mole equivalent in an ether (e.g., THF) under basic conditions, such as in the presence of a lithium diisopropylamide in an mount of 1 to 1.5 mole equivalent at a temperature of from -78°C to room temperature, or in the presence of sodium hexamethyldisilanizide at a temperature of from 0°C to under heating with reflux, followed by removing the protecting groups from the product to give the compound of the formula (55).

The leaving groups represented by G¹ or G² may be a chlorine atom, a bromine atom, etc.

The protecting group for hydroxy group may be any one which does not disturb the reaction, and preferably one is a silyl group such as tert-butyldimethylsilyl group.

The deprotection may be carried out, for example, by the method disclosed in PROTECTIVE GROUPS IN ORGANIC SYNTHESIS, 3rd ed., JOHN WILEY & SONS, INC.: New York (1999).

The compound of the formula (5) or the compound of the formula (55) thus obtained is reacted in a solvent with trifluoromethanesulfonic anhydride in an amount of 2 to 5 mole equivalents in the presence of a base in an amount of 2 to 5 mole equivalents at a temperature of from -30°C to -10°C to give the compound of the formula (6), and without isolating the compound of the formula (6), the compound of the formula (7) in an amount of 1 to 3 mole equivalents and a base in an amount of 1 to 3 mole equivalents are added to the reaction mixture to give the compound of the formula (8).

The solvent may be any solvent which does not disturb the reaction, and may be ethers (e.g., ethyl ether, dimethoxyethane, isopropyl ether, tetrahydrofuran, dioxane, etc.), and nitriles (e.g., acetonitrile, isobutyronitrile, etc.).

The base is preferably a tertiary amine such as triethylamine, etc.

(B) The piperidinecarbonitrile having no substituent at the 1-position, and the piperidinecarbonitrile having a substituent at the 1-position, said substituent being an aromatic group or a substituted aromatic group represented by R¹³, may be prepared as follows. The substituent of the substituted aromatic group represented by R¹³ is a fluorine atom, a trifluoromethyl group, a methylenedioxy group, a lower alkyl group, a lower alkoxy group, a lower alkoxy group being substituted by one or more fluorine atoms, a benzyloxy group, a di-lower alkylamino group, etc. 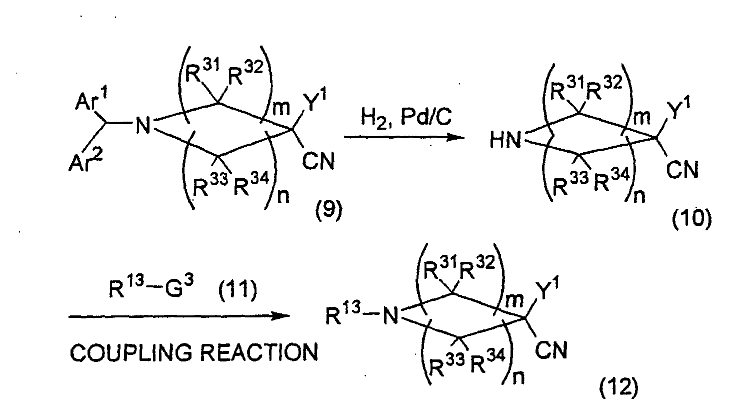 (wherein m, n, R³¹, R³², R³³, R³⁴, Y¹ and R¹³ are as defined above; G³ is a leaving group; Ar¹ is a substituted or unsubstituted phenyl group; Ar² is a hydrogen atom or a substituted or unsubstituted phenyl group; the substituent is a lower alkyl group or a lower alkoxy group, etc.)

Among the compounds of the formula (8) obtained in the above Process (A), the compound of the formula (9) is subjected to hydrogenation in a conventional manner to give the compound of the formula (10).

Subsequently, the compound of the formula (10) is subjected to coupling reaction with the compound of the formula (11) using a palladium catalyst to give the compound of the formula (12).

The coupling reaction may be carried out in the presence of sodium tert-butylate using a palladium (0) catalyst such as tris(dibenzylideneacetone)dipalladium or tetrakis(triphenylphosphine)-palladium, etc., and a phosphorous ligand such as 2,2'-bis(diphenylphosphino)-1,1'-binaphthyl, 1,1'-bis(diphenylphosphino)ferrocene, etc.

The solvent may be any solvent which does not disturb the reaction, but the reaction may preferably proceed when an ether such as tetrahydrofuran, etc. is used.

The leaving group represented by G³ is preferably a iodine atom or a bromine atom.

(C) The present compound of the formula (15) is prepared from the compound of the formula (8) or the compound of the formula (12) obtained in the above Process (A) and (B), respectively, for example, by the following Process. 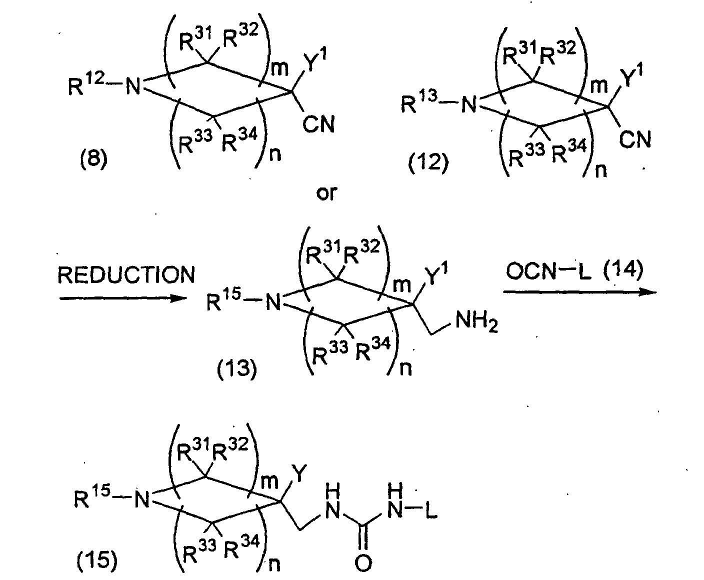 (wherein m, n, R³¹, R³², R³³, R³⁴, R³⁵, R³⁶, L, Y, Y¹, R¹² and R¹³ are as defined above; R¹⁵ is an alkyl group, a substituted alkyl group, an alkenyl group, a substituted alkenyl group, an alkynyl group, a substituted alkynyl group, a cycloalkyl group, a substituted cycloalkyl group, an aromatic group, or a substituted aromatic group, and the substituent of these groups may be the same ones as those for R¹² as mentioned above)

The compound of the formula (8) or the compound of the formula (12) obtained in the above Process (A) or (C) may be converted into the compound of the formula (13) by reduction with lithium aluminum hydride in a conventional manner.

Subsequently, the compound of the formula (13) is reacted with the compound of the formula (14) usually in a solvent at a temperature of from 0°C to 120°C, preferably at a temperature of from room temperature to a boiling point of the solvent, followed by the conversion of substituents and the removal of protecting groups, if necessary, to advantageously give the compound of the formula (15).

The solvent may be any solvent which does not disturb the reaction, and may be ethers (e.g., ethyl ether, dimethoxyethane, isopropyl ether, tetrahydrofuran, dioxane, etc.), aromatic hydrocarbons (e.g., benzene, toluene, xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, dimethylsulfoxide, etc.

(D) In addition, the compound of the present invention of the following formula (64) having a substituent other than an oxo group on the carbon atom attached to the amino group may be prepared from the compound of the formula (8) or the formula (12) obtained in the above Process (A) or (B), respectively, for example, by the following Process. 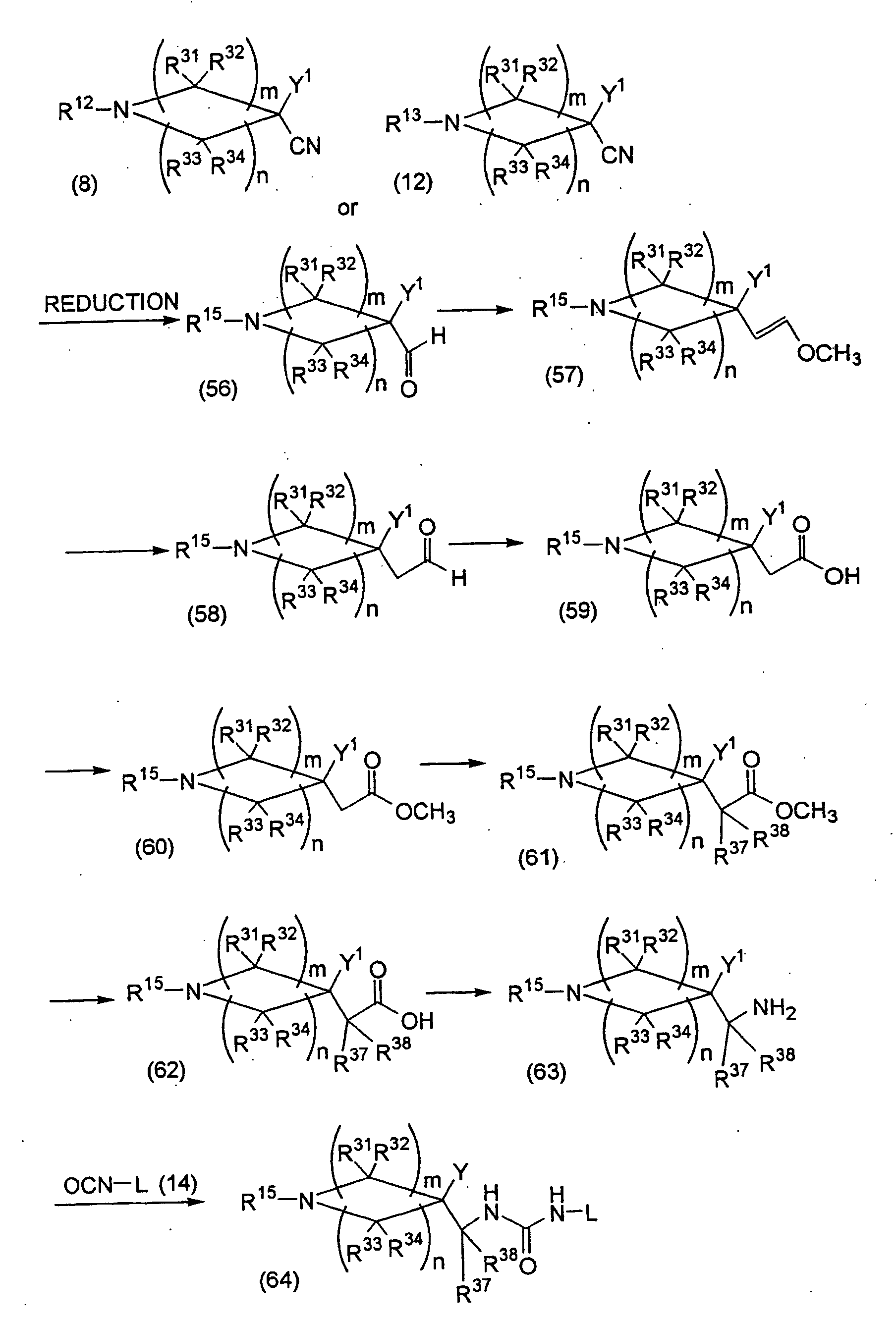 (wherein m, n, R³¹, R³², R³³, R³⁴, R³⁵, R³⁶, L, Y, Y¹, R¹², R¹³ and R¹⁵ are as defined above; R³⁷ and R³⁸ are the same or different, and selected, if both of R³⁷ and R³⁸ exist, independently from a hydrogen atom, an alkyl group, and a substituted alkyl group).

The compound of the formula (8) or the compound of the formula (12) obtained in the above Process (A) or (C) is subjected to reduction using diisobutyl aluminum hydride (DIBAL) in an amount of 1 to 1.5 equivalent in a halogenated hydrocarbon solvent (e.g., methylene chloride) at a temperature of from under ice-cooling to room temperature to give the compound of the formula (56).

Subsequently, the compound of the formula (56) is reacted with a Wittig reagent such as (methoxymethyl)triphenylphosphonium chloride in an amount of 1 to 1.5 equivalent in an ether (e.g., tetrahydrofuran) in the presence of a strong base such as sodium amide (NaNH₂) in an amount of 1 to 1.5 equivalent at a temperature of from -78°C to room temperature to give the compound of the formula (57).

Then, the resultant compound of the formula (57) is reacted in a mixed solvent of a halogenated hydrocarbon solvent such as methylene chloride and trifluoroacetic acid (1:1) at a temperature of from under ice-cooling to room temperature to give the compound of the formula (58).

The compound of the formula (58) is oxidized with an oxidizing agent such as potassium permanganate (KMnO₄) in an amount of 1 to 3 equivalents in a halogenated hydrocarbon solvent such as methylene chloride or chloroform at a temperature of from room temperature to 40°C to give the compound of the formula (59).

The compound of the formula (59) may be converted into the compound of the formula (60), for example, by a method of reacting with methyl iodide in an amount of 1 to 1.5 equivalent in the presence of a base such as potassium carbonate in an amount of about 3 equivalents in dimethylformamide at a temperature of from room temperature to under heating with reflux.

The compound of the formula (60) is reacted with the compound of the formula: R³⁷-G¹ in an amount of 1 to 1.5 equivalent in an ether such as tetrahydrofuran in the presence of a strong base such as lithium diisopropyl amide in an amount of 1 to 1.5 equivalent at a temperature of from -78°C to room temperature, followed by reacting with the compound of the formula: R³⁸-G² in an amount of 1 to 1.5 equivalent in the presence of a strong base such as lithium diisopropyl amide in an amount of 1 to 1.5 equivalent to give the compound of the formula (61) (in which G¹, G², R³⁷ and R³⁸ are as defined above).

Further, the compound of the formula (61) is converted into the compound of the formula (62) by hydrolysis with sodium hydroxide in an amount of 1 to 3 equivalents in a mixed solvent of water and ethanol (1:4) at a temperature of from room temperature to under heating with reflux.

The compound of the formula (62) is converted into the compound of the formula (63) by curtius rearrangement, for example, by reacting with diphenylphosphoryl azide in an amount of 1 to 1.5 equivalent and triethylamine in an amount of 1 to 1.5 equivalent in dimethylformamide at a temperature of from ice-cooling to 60°C.

The compound of the formula (63) thus obtained is reacted with the compound of the formula (14) in a same manner as in the above Process (C), if necessary, followed by the conversion of substituents and the removal of protecting groups, to give the compound of the formula (64).

(E) In addition, the compound of the present invention of the formula (68) having an oxo group on the carbon atom attached to the amino group may be prepared from the compound of the formula (8) or the compound of the formula (12) obtained in the above Process (A) or (B), respectively, by the following Process. 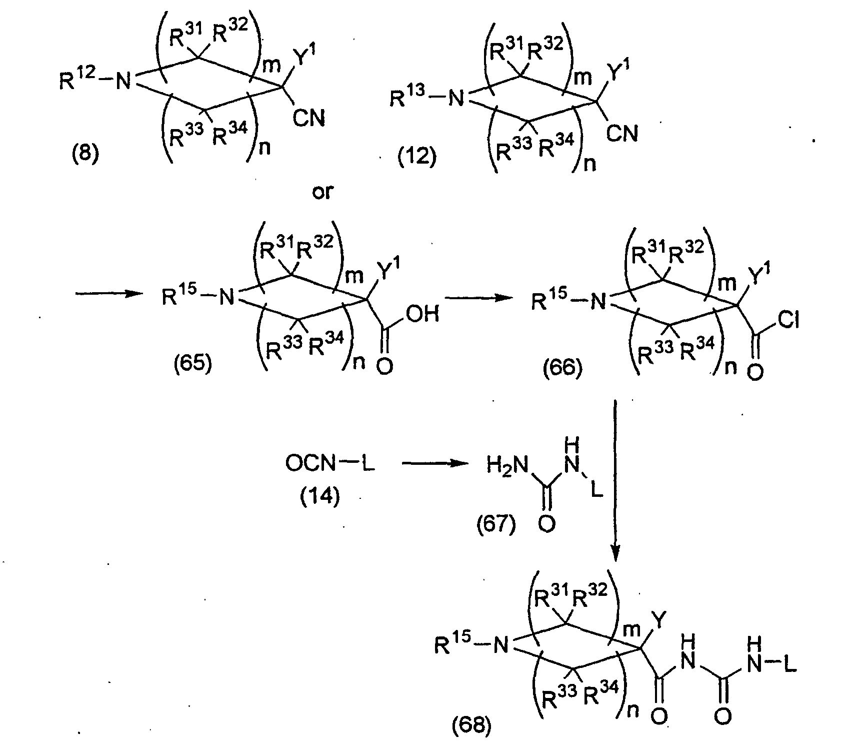 (wherein m, n, R³¹, R³², R³³, R³⁴, L, Y, Y¹, R¹², R¹³ and R¹⁵ are as defined above)

The compound of the formula (8) or the compound of the formula (12) obtained in the above Process (A) or (C), respectively, is reacted in a 48 % aqueous hydrobromic acid solution at a temperature of from room temperature to under heating with reflux to give the compound of the formula (65).

Then, the compound of the formula (65) is reacted with oxalyl chloride ((COCl)₂) in an amount of 2 to 3 equivalents in a halogenated hydrocarbon solvent (e.g., methylene chloride, etc.) at a temperature of from 0°C to room temperature to give the compound of the formula (66).

On the other hand, the compound of the formula (14) is reacted in a mixed solvent of a 28 % aqueous ammonia in 1,4-dioxane (1:4) at a temperature of from 0°C to room temperature to give the compound of the formula (67).

The compound of the above formula (66) is reacted with the compound of the formula (14) in a halogenated hydrocarbon solvent (e.g., methylene chloride) at a temperature of from room temperature to under heating with reflux in the presence of a base such as triethylamine in an amount of 1 to 3 equivalents, if necessary, followed by the conversion of substituents and the removal of protecting groups to give the compound of the formula (68).

(F) In addition, the compound of the present invention of the formula (15') may be prepared by the following Process. 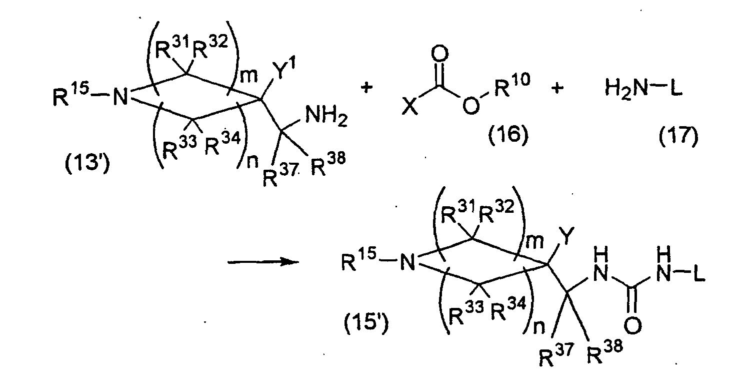 (wherein m, n, R³¹, R³², R³³, R³⁴, R³⁷, R³⁸, L, Y, Y¹ and R¹⁵ are as defined above; X is a chlorine atom or a bromine atom; R¹⁰ is a lower alkyl group or a substituted phenyl group)

The compound of the formula (13') is reacted with the compound of the formula (16) at a temperature of from 0°C to 80°C to a carbamic acid ester, which is reacted with the compound of the formula (17) at a temperature of from room temperature to a boiling point of the solvent, or at a temperature of from room temperature to 100°C, if necessary, followed by the conversion of substituents and the removal of protecting groups to give the compound of the present invention of the formula (15').

The compound of the formula (16) includes, for example, methyl chlorocarbonate, ethyl chlorocarbonate, isobutyl chlorocarbonate, phenyl chlorocarbonate, p-nitrophenoyl chlorocarbonate, etc.

The reaction is usually carried out in a solvent, and the solvent may be any solvent which does not disturb the reaction, for example, ethers (e.g., ethyl ether, isopropyl ether, tetrahydrofuran, dioxane, etc.), aromatic hydrocarbons (e.g., benzene, toluene, xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, dimethylsulfoxide, etc.

Alternatively, in a similar manner, the compound of the formula (17) and the compound of the formula (16) are reacted first, then, the resultant product is reacted with the compound of the formula (13'), if necessary, followed by the conversion of substituents and the removal of protecting groups to give the compound of the formula (15').

(G) The compound of the present invention may also be prepared by the following Process, which is different from the above Processes (A) to (D). 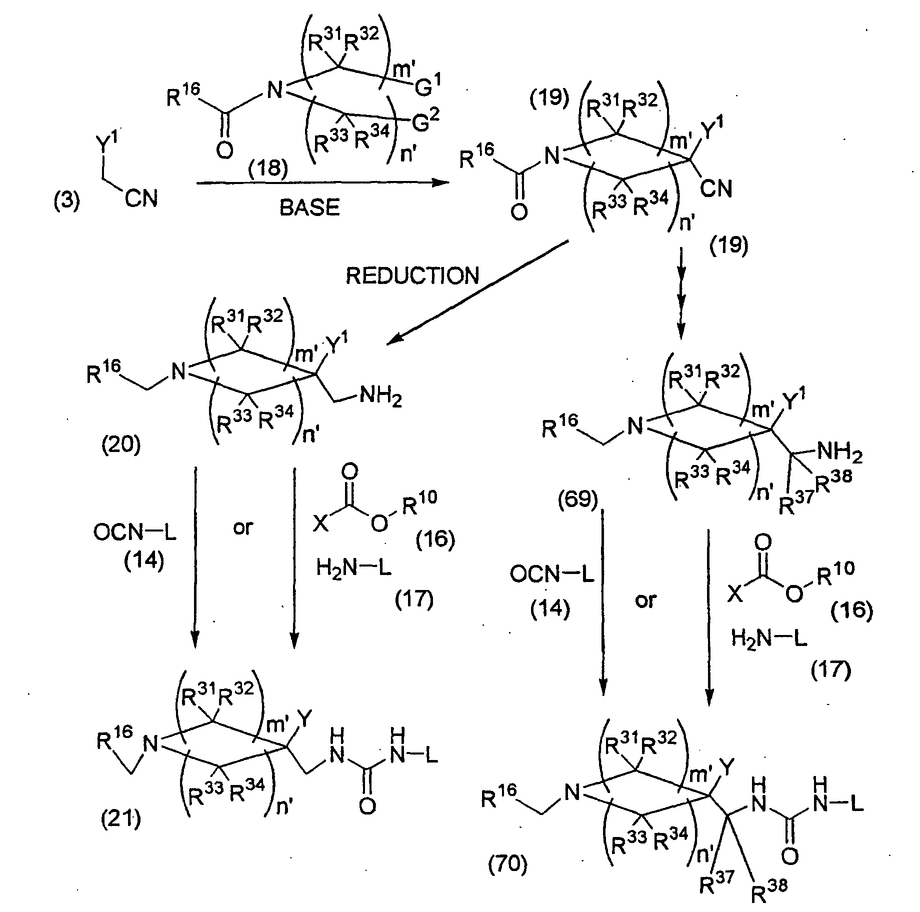 (wherein m', n', R³¹, R³², R³³, R³⁴, R³⁷, R³⁸, L, Y, Y¹, G¹, X, and R¹⁰ are as defined above; R¹⁶ is an alkyl group, a substituted alkyl group, an alkenyl group, a substituted alkenyl group, an alkynyl group, a substituted alkynyl group, a cycloalkyl group, a substituted cycloalkyl group, an aromatic group, or a substituted aromatic group; the substituents on these groups are ones as defined for R¹² as mentioned above)

The compound of the formula (3) is reacted with the compound of the formula (18) in an amount of 1 to 2 equivalents in the presence of a base (e.g., sodium hydride, etc.) in an amount of 1 to 3 equivalents at a temperature of from room temperature to a boiling point of the solvent to give the compound of the formula (19).

The leaving group represented by G¹ or G² may be a chlorine atom, a bromine atom, etc.

The reaction is usually carried out in a solvent, and the solvent may be any one which does not disturb the reaction, for example, ethers (e.g., ethyl ether, dimethoxyethane, isopropyl ether, tetrahydrofuran, dioxane, etc.), dimethylsulfoxide, etc.

The compound of the formula (19) thus obtained is treated with lithium aluminum hydride in a usual manner for reduction of the nitrile group and the amido group to give the compound of the formula (20). Then, the compound of the formula (20) is converted into the compound of the present invention of the formula (21) in a similar manner to that of the preparation of the compound of the formula (15) or the compound of the formula (15') from the compound of the formula (13) or the compound of the formula (13') in the above Process (C) or (F).

Alternatively, the compound of the formula (69) is prepared from the compound of the formula (19) in a similar manner to the above Process (D), and the compound of the formula (69) is further reacted with the compound of the formula (14) as mentioned above, or reacted with the compound of the formula (16) and the compound of the formula (17), if necessary, followed by the conversion of substituents and the removal of protecting groups to give the compound of the present invention of the formula (70).

(H) Among the compounds of the formula (15) and the formula (15') obtained by the above Processes, the compound of the formula (22) is converted in the compound of the formula (23) by a conventional hydrogenation. 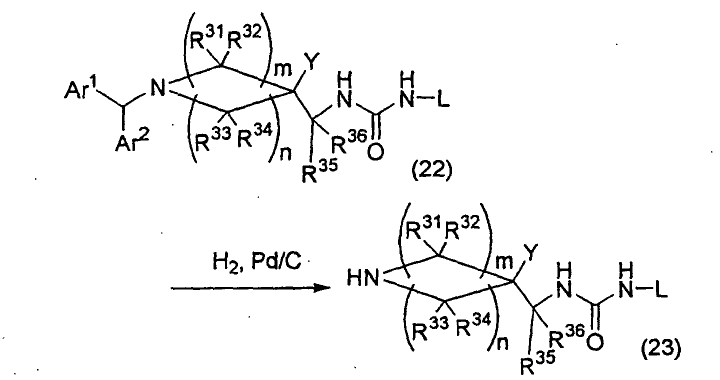 (wherein m, n, R³¹, R³², R³³, R³⁴, R³⁵, R³⁶, L, Y, Ar¹ and Ar² are as defined above)

(I) The following derivatives are obtained from the compound of the formula (23) obtained in the above. 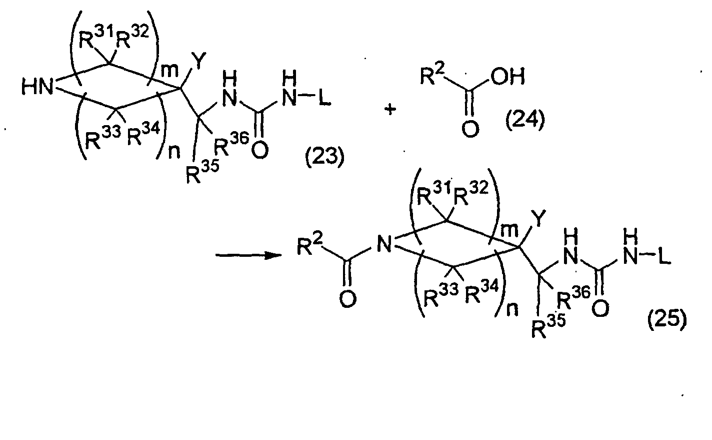 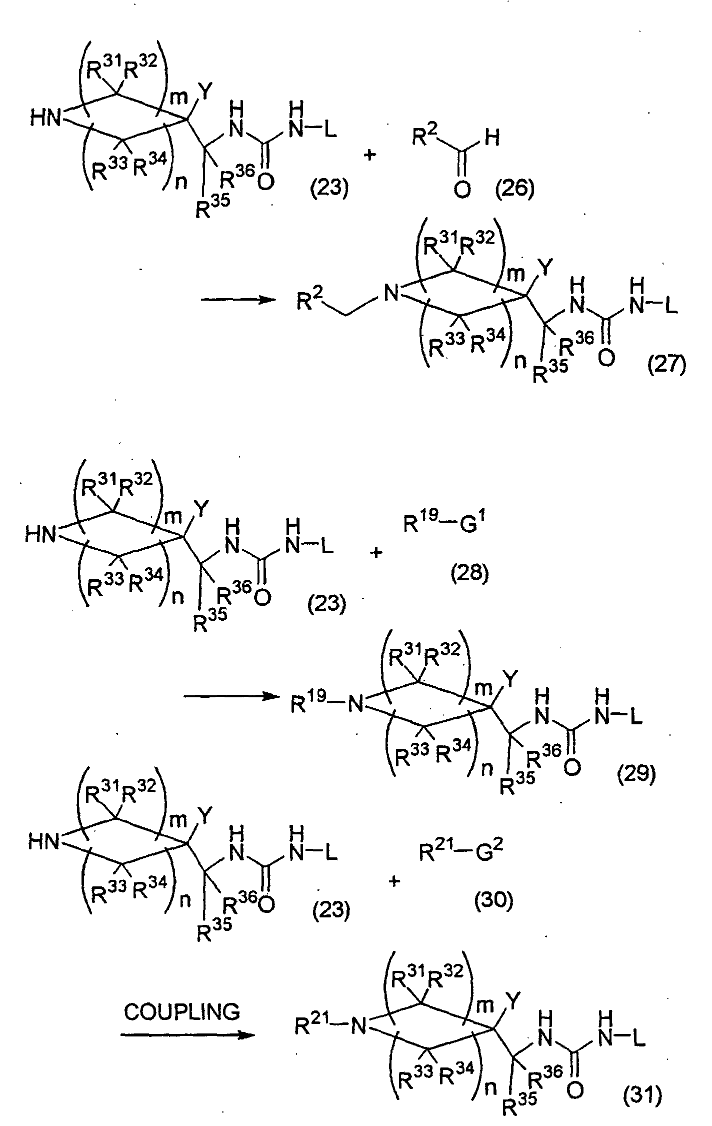 (wherein m, n, R³¹, R³², R³³, R³⁴, R³⁵, R³⁶, L, G¹, G², Y and R² are as defined above; R¹⁹ is an alkyl group, a substituted alkyl group, an alkenyl group, a substituted alkenyl group, an alkynyl group, a substituted alkynyl group, a cycloalkyl group, or a substituted cycloalkyl group; R²¹ is an aromatic group or a substituted aromatic group)

The compound of the formula (23) and the compound of the formula (24) are condensed in a solvent using a condensing agent at a temperature of from 0°C to 100°C, preferably at a temperature of from 0°C to 60°C, if necessary, followed by the conversion of substituents and/or the deprotection to give the compound of formula (25).

The condensing agent includes, for example, dicyclohexylcarbodiimide (DCC), 1,1'-carbonyldiimidazole, diethyl cyanophosphate (DEPC), 1-ethyl-3-(3'-dimethylaminopropyl)carbodiimide hydrochloride (WSC), etc.

The reaction may advantageously proceed by addition of a base in an amount of 1 to 5 mole equivalents, preferably in an amount of 1 to 3 mole equivalents, to the amount of the compound of the formula (23). The base may be a tertiary amine such as triethylamine, diisopropylethylamine, or pyridine, etc.

The solvent may be any solvent which does not disturb the reaction, for example, ethers (e.g., diethyl ether, dimethoxyethane, tetrahydrofuran, dioxane, etc.), aromatic hydrocarbons (e.g., benzene, toluene, xylene, etc.), esters (e.g., ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), and amides (e.g., N,N-dimethylformamide, N,N-dimethylacetamide, etc.).

Alternatively, the compound of the formula (24) is converted into a reactive derivative thereof, which is further reacted with the compound of the formula (23) in a solvent at a temperature of from -10°C to 120°C, preferably at a temperature of from 0°C to 60°C, if necessary, followed by the conversion of substituents and/or the deprotection to give the compound of the formula (25).

The reactive derivative of the compound of the formula (24) may be, for example, an acid chloride, an acid bromide, an acid anhydride, or a mixed acid anhydride with methyl carbonate, ethyl carbonate, etc., and the reaction may advantageously proceed by addition of a base in an amount of 1 to 5 mole equivalents, preferably in an amount of 1 to 3 mole equivalents. The base may be a tertiary amine (e.g., triethylamine, etc.), pyridine, an alkali metal carbonate (e.g., sodium carbonate, potassium carbonate, etc.), and an alkali metal hydrogen carbonate (e.g., sodium hydrogen carbonate, etc.). The solvent may be any solvent which does not disturb the reaction, for example, ethers (e.g., diethyl ether, dimethoxyethane, tetrahydrofuran, dioxane, etc.), aromatic hydrocarbons (e.g., benzene, toluene, xylene, etc.), esters (e.g., ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), and amides (e.g., N,N-dimethylformamide, N,N-dimethylacetamide, etc.).

In addition, the compound of formula (23) and the compound of the formula (26) in an amount of 1 to 5 equivalents are treated with a reducing agent in an amount of 1 to 5 equivalents at a temperature of from 0 to 50°C in a lower alcohol (e.g., methanol, ethanol, etc.), if necessary, followed by the conversion of substituents and/or the deprotection to give the compound of the formula (27). The reducing agent includes, for example, sodium borohydride (NaBH₄), sodium cyanoborohydride (NaBH₃CN), sodium triacetoxyborohydride (NaB(O₂CCH₃)₃H), etc.

The compound of the formula (23) is reacted with the compound of the formula (28) in a solvent, if necessary, followed by the conversion of substituents and/or the deprotection to give the compound of the formula (29).

The reaction is usually carried out in solvent at a temperature of from 0°C to 100°C, preferably at a temperature of from room temperature to 70°C, if necessary, in the presence of a base.

The solvent may be, for example, ethers (e.g., tetrahydrofuran, dioxane, etc.), aromatic hydrocarbons (e.g., benzene, toluene, etc.), ketones (e.g., acetone, 2-butonone, etc.), dimethylformamide, etc., and the base may be, for example, sodium hydride, potassium carbonate, sodium carbonate, triethylamine, etc.

When potassium carbonate or sodium carbonate is used, the yield of the reaction may occasionally be improved by addition of sodium iodide or potassium iodide into the reaction system.

The leaving group represented by G¹ is usually a halogen atom (e.g., chlorine, bromine, iodine, etc.), aromatic sulfonyloxy group (e.g., benzenesulfonyloxy group, p-toluenesulfonyloxy group, etc.) or methanesulfonyloxy group.

In a similar manner to the conversion from the compound of the formula (10) into the compound of the formula (12), the compound of the formula (23) is coupled with the compound of the formula (30), if necessary, followed by the conversion of substituents and/or the deprotection to give the compound of the formula (31).

(J) The compound wherein the group Y is a substituted phenyl group, and the substituent thereof is a group of the formula :-O-E-A may be prepared by the following Process.

The compound of the formula (35) is prepared from the compound of the formula (32), which is obtained in the above Processes, by the following Process. 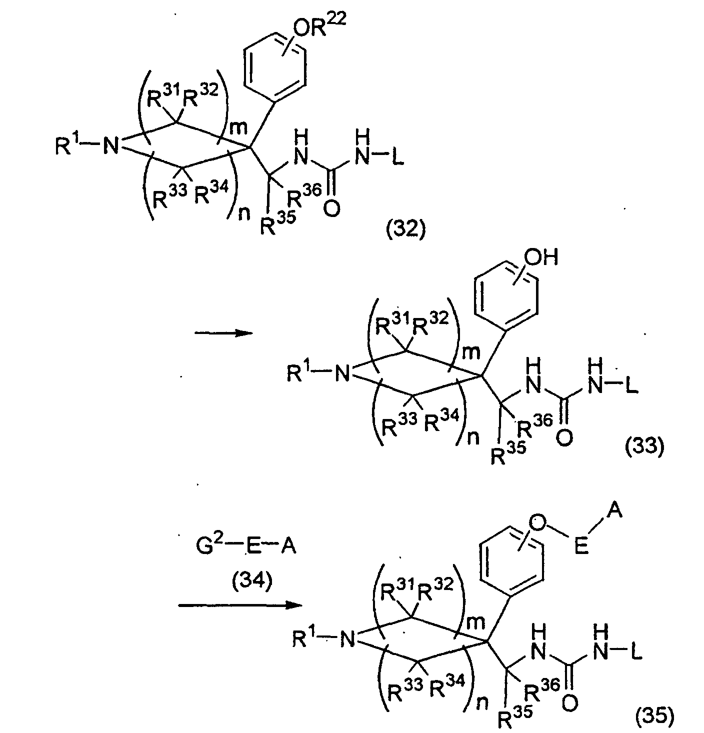 (wherein m, n, R³¹, R³², R³³, R³⁴, R³⁵, R³⁶, L, G², E and A are as defined above; R²² is a methyl group or a benzyl group; R¹ is the same groups for the above R except for a hydrogen atom)

The protecting group R²² of the compound of the formula (32) is removed by the method disclosed in the literature (e.g., PROTECTIVE GROUPS IN ORGANIC SYNTHESIS, 3rd ed., JOHN WILEY & SONS, INC.: New York (1999)) to give the compound of the formula (33).

Then, the compound of the formula (33) is reacted with the compound of the formula (34) in a solvent, if necessary, followed by the conversion of substituents and/or the deprotection to give the compound of the formula (35).

The reaction is usually carried out in a solvent at a temperature of from 0°C to 100°C, preferably at a temperature of from room temperature to 70°C in the presence of a base.

The solvent may be ethers (e.g., tetrahydrofuran, dioxane, etc.), aromatic hydrocarbons (e.g., benzene, toluene, etc.), ketones (e.g., acetone, 2-butanone, etc.), dimethylformamide, etc. The base includes, for example, sodium hydride, potassium carbonate, sodium carbonate, triethylamine, etc.

When potassium carbonate or sodium carbonate is used, the yield of the reaction may occasionally be improved by addition of potassium iodide or sodium iodide into the reaction system.

The leaving group represented by G² is usually a halogen atom (e.g., chlorine, bromine, iodine, etc.), aromatic sulfonyloxy group (e.g., benzenesulfonyloxy group, p-toluenesulfonyloxy group, etc.), or methanesulfonyloxy group.

The starting compounds used in the above-mentioned each Process may be known compounds or may be prepared from a conventional known compound by a conventional method known by the ordinary skilled person in this art or a modified method thereof.

Each compound obtained in the above-mentioned Processes may be isolated and purified by a conventional isolation method such as recrystallization, a conventional purification method using chromatography, solvent extraction method, or reprecipitation.

The product obtained in any one of the Processes may be in the form of an acid addition salt thereof or a free base, due to the reaction conditions. These products may be converted into a desired acid addition salt or a free base by a conventional method.

### EXAMPLES

The present invention will be illustrated in more detail by the following Reference Examples and Examples, and should not be construed to be limited thereto.

### Reference Example 1

### Preparation of 1-benzoyl-4-(3-methoxyphenyl)piperidine-4-carbonitrile

To a solution of sodium hydride (1.36 g, 34 mmol) in dimethylsulfoxide (DMSO, 30 ml) is added a solution of (3-methoxyphenyl)-acetonitrile (1.90 ml, 13.6 mmol) and N,N-bis(2-chloroethyl)benzamide (3.17 g, 13.6 mmol) in ether (10 ml) at room temperature, and the mixture is stirred overnight. To the reaction mixture is added water, and the mixture is extracted twice with ethyl acetate. The organic layer is washed twice with water, dried over anhydrous magnesium sulfate, and the solvent is evaporated under reduced pressure. Then, the residue is purified by silica gel chromatography and crystallized from hexane to give the title compound (2.26 g) as white crystals.
¹H-NMR δ (CDCl₃):1.95-2.21 (4H, brd), 3.28 (1H, brd), 3.54 (1H, brd), 3.85 (3H, s), 3.93 (1H, brd), 4.94 (1H, brd), 6.87-6.91 (1H, m), 7.01-7.07 (2H, m), 7.32-7.39 (1H, m), 7.43 (5H, s).

### Reference Example 2

### Preparation of [1-benzyl-4-(3-methoxyphenyl)piperidin-4-yl]methylamine

To a suspension of lithium aluminum hydride (1.18 g, 31.2 mmol) in ether (50 ml) is added ice-cold conc. sulfuric acid (1.73 ml, 31.2 mmol). To the mixture is added a solution of 1-benzoyl-4-(3-methoxyphenyl)piperidine-4-carbonitrile (2.00 g, 6.24 mmol) in ether (10 ml) at room temperature, and the mixture is stirred for 15 minutes. The mixture is heated under reflux for 2 hours, and thereto is added aqueous ammonia. The mixture is filtered on Celite, and the solvent is evaporated under reduced pressure to give the title compound (1.94 g) as colorless oil.
¹H-NMR δ (CDCl₃): 1.76-1.84 (2H, m), 2.16-2.25 (4H, m), 2.60-2.62 (2H, m), 2.74 (2H, s), 3.41 (2H, s), 3.82 (3H, s), 6.75-6.79 (1H, m), 6.86-6.91 (2H, m), 7.23-7.31 (6H, m).

### Reference Example 3

### Preparation of [1-phenyl-4-(3-hydroxyphenyl)piperidin-4-yl]methylamine

To a solution of [1-phenyl-4-(3-methoxyphenyl)piperidin-4-yl]-methylamine (200 mg, 0.675 mmol) in methylene chloride (20 ml) is added dropwise boron tribromide (0.058 ml, 0.609 mmol) under ice cooling over a period of 10 minutes. The mixture is warmed to room temperature, and stirred overnight. To the reaction solution is added ice-cold methanol, and the mixture is concentrated under reduced pressure. To the reaction solution is added ether, and the mixture is extracted twice with 3N aqueous hydrochloric acid solution. The aqueous layer is basified with aqueous ammonia, and dried over anhydrous magnesium sulfate, and the solvent is evaporated under reduced pressure to give the title compound (144 mg) as oil.
¹H-NMR δ (DMSO-d₆): 1.80-1.88 (2H, m), 2.08-2.13 (2H, m), 2.59 (2H, s), 2.81-2.88 (2H, m), 3.32-3.37 (4H, m), 6.57-6.60 (1H, m), 6.66-6.79 (3H, m), 6.86-6.89 (2H, m), 7.10-7.17 (3H, m), 9.24 (1H, brdm).

### Reference Example 4

### Preparation of 4-hydroxy-2-(2-hydroxyethyl)-(3-methoxyphenyl)butanenitrile

To a solution of sodium hydride (1.79 g, 44.8 mmol) in dimethylsulfoxide (DMSO, 60 ml) is added a solution of (3-methoxyphenyl)acetonitrile (2.50 ml, 17.9 mmol) and 2-bromoethyl tert-butyldimethylsilyl ether (9.22 ml, 43.0 mmol) in ether (20 ml) at room temperature, and the mixture is stirred overnight. To the mixture is added water, and the mixture is extracted twice with ether. The organic layer is washed three times with water, dried over anhydrous magnesium sulfate, and the solvent is evaporated under reduced pressure. Then, the reaction mixture is dissolved in tetrahydrofuran (THF, 100 ml), and thereto is added tetrabutylammonium fluoride (TBAF, 12.2 g, 46.5 mmol), and the mixture is stirred at room temperature overnight. Water is added to the mixture, and the mixture is extracted twice with ethyl acetate. The organic layer is washed twice with water, dried over anhydrous magnesium sulfate, and the solvent is evaporated under reduced pressure. The residue is purified by silica gel chromatography to give the title compound (2.91 g) as colorless oil. ¹H-NMR δ (DMSO-d₆): 1.97-2.02 (4H, m), 3.12-3.22 (2H, m), 3.32-3.45 (2H, m), 3.76 (3H, s), 4.59 (2H, t, J = 5.13), 6.87-7.03 (3H, m), 7.31-7.36 (1H, m).

### Reference Example 5-1

### Preparation of 1-(4-fluorophenyl)-4-(3-methoxyphenyl)piperidine-4-carbonitrile

To a solution of 4-hydroxy-2-(2-hydroxyethyl)-(3-methoxyphenyl)butanenitrile (70 mg, 0.195 mmol) in acetonitrile (6 ml) are successively added trifluoromethanesulfonic anhydride (0.11 ml, 0.626 mmol) and triethylamine (0.080 ml, 0.626 mmol) at a temperature of -30°C to -20°C, and the mixture is stirred for 15 minutes. To the mixture are added 4-fluoroaniline (0.037 ml, 0.387 mmol) and triethylaniline (0.066 ml, 0.387 mmol) at a temperature of -30°C to -20°C, and the mixture is slowly warmed to room temperature and stirred for 2 hours. The reaction is quenched by addition of water, and the mixture is extracted with ether. The organic layer is washed twice with water, dried over anhydrous magnesium sulfate, and the solvent is evaporated under reduced pressure. The resultant is purified by preparative thin layer chromatography (preparative TLC) to give the title compound (53.5 mg) as colorless oil.
¹H-NMR δ (DMSO-d₆): 2.12-2.27 (4H, m), 2.88-2.97 (2H, m), 3.72-3.78 (5H, m), 6.93-6.97 (1H, m), 7.03-7.15 (6H, m), 7.35-7.40 (1H, m).

### Reference Example 5-2

### Preparation of 1-benzhydrile-4-(3-methoxyphenyl)piperidine-4-carbonitrile

The title compound is prepared in a similar manner to Reference Example 5-1.
¹H-NMR δ (DMSO-d₆): 2.08 (4H, m), 2.18-2.26 (2H, m), 2.88-2.93 (2H, m), 3.78 (3H, s), 4.45 (1H, s), 6.91-6.94 (1H, m), 7.03-7.05 (1H, m), 7.09-7.12 (1H, m), 7.16-7.21 (2H, m), 7.27-7.37 (5H, m), 7.44-7.47 (4H, m).

### Reference Example 6

### Preparation of 4-(3-methoxyphenyl)piperidine-4-carbonitrile

A suspension of 1-benzhydrile-4-(3-methoxyphenyl)piperidine-4-carbonitrile (34.3 mg, 0.090 mmol), acetic acid (0.005 ml, 0.090 mmol) and 10 % palladium hydroxide (3.4 mg) in methanol (20 ml) is stirred under hydrogen atmosphere for 3 hours. The mixture is filtered on Celite, and the solvent was evaporated under reduced pressure. To the resultant is added a saturated aqueous sodium hydrogen carbonate solution, and the mixture is extracted with ethyl acetate. The extract is washed once with a saturated aqueous sodium hydrogen carbonate solution, dried over anhydrous magnesium sulfate. The solvent is evaporated under reduced pressure, and the residue is purified by preparative TLC to give the title compound (12.9 mg) as oil.
¹H-NMR δ (DMSO-d₆): 1.86-2.06 (4H, m), 2.79-2.87 (2H, m), 3.07-3.11 (2H, m), 3.77 (3H, s), 6.92-6.96 (1H, m), 7.00-7.08 (2H, m), 7.33-7.39 (1H, m).

### Reference Example 7-1

### Preparation of 1-(3-pyridyl)-4-(3-methoxyphenyl)piperidine-4-carbonitrile

To a solution of tris(dibenzylideneacetone)dipalladium (Pd₂(dba)₃, 14.0 mg, 10 mol %) and 2,2'-bis(diphenylphosphino)-1,1'-binaphthyl (BINAP, 17.0 mg, 20 mol %) in tetrahydrofuran (THF, 100 ml) are added 3-iodopyridine (34 mg, 0.166 mmol), 4-(3-methoxyphenyl)piperidine-4-carbonitrile (30 mg, 0.139 mmol), sodium tert-butylato (47 mg, 0.417 mmol) at room temperature, and the mixture is heated under reflux for 5 hours. The reaction is quenched by addition of a saturated brine, and the mixture is extracted with ethyl acetate, and the organic layer is washed twice with a saturated brine. The extract is dried over anhydrous magnesium sulfate and concentrated under reduced pressure. The resultant is purified by preparative TLC to give the title compound (28.0 mg) as oil.
High performance liquid chromatography/Mass spectroscopy
m/z 294.1 (M+H)
Retention time: 0.22 min., 2.86 min.

### Reference Example 7-2

### Preparation of 1-[3-(benzyloxy)pyridin-2-yl]-4-(3-methoxyphenyl)-piperidine-4-carbonitrile

The title compound is prepared in a similar manner to Reference Example 7-1.
High performance liquid chromatography/Mass spectroscopy
m/z 400.3 (M+H)
Retention time: 3.17 min.

### Reference Example 8

### Preparation of 3-(benzyloxy)-2-bromopyridine

To a solution of 2-bromo-3-pyridinol (1.74 g, 10.0 mmol) and potassium carbonate (4.15 g, 30.0 mmol) in dimethylformamide (DMF, 150 ml) is added benzyl bromide (1.49 ml, 12.5 mmol) at room temperature, and the mixture is warmed to 55°C and stirred for 3 hours. The reaction is quenched by addition of water, and the mixture is extracted with ethyl acetate. The organic layer is washed twice with water, dried over anhydrous magnesium sulfate, and evaporated under reduced pressure. The residue is purified by silica gel chromatography to give the title compound (2.61 g) as oil.
¹H-NMR δ (DMSO-d₆): 7.33-7.49 (6H, m), 7.58-7.61 (1H, m), 7.95-7.98 (1H, m).

### Example 1-1

### Preparation of N-{[1-benzyl-4-(3-methoxyphenyl)piperidin-4-yl]methyl}-N'-(2,6-diisopropylphenyl)urea

To a solution of 2,6-diisopropyl isocyanate (1.27 g, 6.25 mmol) in tetrahydrofuran (THF, 100 ml) is added [1-benzyl-4-(3-methoxyphenyl)piperidin-4-yl]methylamine (1.94 g, 6.25 mmol) at room temperature. The mixture is concentrated under reduced pressure, and the resultant is purified by silica gel chromatography to give the title compound (3.02 g) as amorphous.
¹H-NMR δ (CDCl₃): 1.05 (12H, brd), 1.78-1.81 (2H, m), 1.95 (2H, m), 2.26 (2H, m), 2.53 (2H, m), 3.05-3.15 (2H, m), 3.31 (2H, d, J = 5.49), 3.40 (2H, s), 3.71 (3H, s), 5.63 (1H, s), 6.53-6.62 (3H, m), 6.99 (1H, t, J = 7.32), 7.07 (2H, d, J = 7.86), 7.23-7.28 (7H, m).

### Example 1-2

### Preparation of N-{[1-phenyl-4-(3-methoxyphenyl)piperidin-4-yl]methyl}-N'-(2,6-diisopropylphenyl)urea

The title compound is prepared in a similar manner to Example 1-1.
¹H-NMR δ (CDCl₃): 1.06-1.14 (12H, brd), 1.85-1.94 (2H, m), 2.10-2.15 (2H, m), 2.94-3.02 (2H, m), 3.09-3.18 (2H, m), 3.30-3.36 (4H, m), 3.70-3.79 (4H, m), 5.83 (1H, s), 6.55-6.63 (3H, m), 6.76-6.88 (3H, m), 6.99-7.30 (7H, m)

### Example 2

### Preparation of N-{[4-(3-methoxyphenyl)piperidin-4-yl]methyl}-N'-(2,6-diisopropylphenyl)urea

A suspension of 50 % (w/w) palladium on carbon (200 mg) and N-{[1-benzyl-4-(3-methoxyphenyl)piperidin-4-yl]methyl}-N'-(2,6-diisopropylphenyl)urea (2.02 g, 3.93 mmol) in methanol (30 ml) is stirred under hydrogen atmosphere at room temperature for 4 hours. To the reaction mixture is added 10 % hydrochloric acid/methanol (2 ml), and the mixture is filtered on Celite, and the solvent is evaporated under reduced pressure. The resultant is crystallized from ether to give the hydrochloride of the title compound (1.93 g) as white crystals. ¹H-NMR δ (DMSO-d₆): 1.06 (12H, d, J = 6.75), 1.90-2.04 (4H, brd), 2.74 (2H, brd), 2.97-3.06 (4H, brdm), 3.33 (2H, brd), 3.77 (3H, s), 5.87 (1H, brd), 6.83-6.96 (3H, brdm), 7.04-7.31 (4H, brdm), 7.47 (1H, brd).

### Example 3

### Preparation of N-{[1-acetyl-4-(3-methoxyphenyl)piperidin-4-yl]methyl}-N'-(2,6-diisopropylphenyl)urea

To N-{[4-(3-methoxyphenyl)piperidin-4-yl]methyl}-N'-(2,6-diisopropylphenyl)urea hydrochloride (500 mg, 1.09 mmol) is added acetyl chloride (1.20 ml, 0.085 mmol) under ice-cooling, and thereto is further added triethylamine (2.40 ml, 0.334 mmol), and the mixture is stirred at room temperature for 2 hours. Water is added to the reaction mixture, and the mixture is extracted twice with ethyl acetate. The organic layer is washed twice with water, dried over anhydrous magnesium sulfate, and the solvent is evaporated under reduced pressure. The resultant is crystallized from ether/hexane to give the title compound (399 mg) as white crystals.
¹H-NMR δ (CDCl₃): 1.08 (12H, brd), 1.65-1.76 (2H, brdm), 1.88-1.99 (2H, brdm), 2.03 (3H, s), 3.08-3.31 (6H, m), 3.54-3.91 (5H, m), 6.00 (1H, s), 6.57-6.67 (4H, m), 7.04-7.11 (3H, m), 7.25-7.30 (1H, m).

### Example 4

### Preparation of N-{[1-ethyl-4-(3-methoxyphenyl)piperidin-4-yl]methyl}-N'-(2,6-diisopropylphenyl)urea

To a suspension of lithium aluminum hydride (16 mg, 0.430 mmol) in tetrahydrofuran (THF, 10 ml) is added N-{[1-acetyl-4-(3-methoxyphenyl)piperidin-4-yl]methyl}-N'-(2,6-diisopropylphenyl)urea (200 mg, 0.430 mmol) at room temperature, and the mixture is stirred overnight. To the mixture is added aqueous ammonia, and the mixture is filtered on Celite. The solvent is evaporated under reduced pressure, and the residue is purified by preparative TLC to give the title compound (45 mg) as white crystal.
High performance liquid chromatography/Mass spectroscopy
m/z 452.1 (M+H)
Retention time: 3.63 min.

### Example 5-1

### Preparation of N-{[1-methyl-4-(3-methoxyphenyl)piperidin-4-yl]methyl}-N'-(2,6-diisopropylphenyl)urea

To a solution of N-{[4-(3-methoxyphenyl)piperidin-4-yl]methyl}-N'-(2,6-diisopropylphenyl)urea (500 mg, 1.09 mmol) in methanol (10 ml) are added a 37 % aqueous formaldehyde solution (176 mg, 2.17 mmol) and sodium cyanoborohydride (NaBH₃CN, 103 mg, 1.64 mmol) under ice-cooling, and the mixture is warmed to room temperature and stirred for 3 hours. Water is added to the reaction mixture, and the mixture is extracted twice with ethyl acetate. The organic layer is washed twice with water, dried over anhydrous magnesium sulfate, and the solvent is evaporated under reduced pressure to give the title compound (481 mg) as white crystals.
¹H-NMR δ (CDCl₃): 1.07 (12H, brd), 1.97-2.14 (2H, brd), 2.40-2.56 (5H, brd), 2.83 (2H, brd), 3.05-3.14 (2H, m), 3.35 (2H, brd), 3.77 (3H, s), 5.90 (1H, brd), 6.54-6.74 (3H, brdm), 7.07-7.28 (5H, m).

### Example 5-2

### Preparation of N-{[1-butyl-4-(3-methoxyphenyl)piperidin-4-yl]methyl}-N'-(2,6-diisopropylphenyl)urea

The title compound is prepared in a similar manner to Example 5-1.
¹H-NMR δ (DMSO-d₆): 0.839 (3H, t, J = 7.14), 1.06 (12H, d, J = 6.78), 1.82 (2H, brd), 1.98 (2H, brd), 2.19 (4H, brd), 2.30 (2H, brd), 2.50 (2H, brd), 2.97-3.06 (2H, m), 3.75 (3H, s), 5.67 (1H, brd), 6.80-7.33 (8H, brdm).

### Example 6-1

### Preparation of N-{[1-phenyl-4-(3-methoxyphenyl)piperidin-4-yl]methyl}-N'-(4-tert-butoxycarbonylamino-2,6-diisopropylphenyl)urea

To a solution of [1-phenyl-4-(3-methoxyphenyl)piperidin-4-yl]-methylamine (300 g, 1.01 mmol) in tetrahydrofuran (THF, 10 ml) is added tert-butyl 3,5-diisopropyl-4-({[(4-nitrophenyl)oxy]carbonyl}amino)-phenylcarbamate (486 mg, 1.06 mmol), and the mixture is stirred overnight. Water is added to the reaction mixture, and the mixture is extracted twice with ethyl acetate. The organic layer is washed twice with water, and dried over anhydrous magnesium sulfate. The solvent is evaporated under reduced pressure, and the residue is purified by silica gel chromatography to give the title compound (522 mg) as colorless amorphous.
¹H-NMR δ (CDCl₃): 1.02-1.13 (12H, m), 1.55 (9H; s), 1.86-1.93 (2H, m), 2.05-2.16 (2H, m), 2.93-3.00 (2H, m), 3.05-3.14 (2H, m), 3.31-3.38 (4H, m), 3.71 (3H, s), 5.76 (1H, s), 6.59-6.87 (7H, m), 7.04-7.26 (5H, m).

### Example 6-2

### Preparation of N-{[1-phenyl-4-(3-hydroxyphenyl)piperidin-4-yl]methyl}-N'-(4-tert-butoxycarbonylamino-2,6-diisopropylphenyl)urea

The title compound is prepared from [1-phenyl 4-(3-hydroxyphenyl)piperidin-4-yl]methylamine in a similar manner to Example 6-1. ¹H-NMR δ (DMSO-d₆): 1.04 (12H, d, J = 6.78), 1.45 (9H, s), 1.89-2.01 (4H, brd), 2.95-3.04 (4H, m), 3.31 (2H, brd), 5.69 (1H, brd), 6.61-6.89 (6H, m), 7.13-7.24 (6H, m), 9.09 (1H, brd), 9.28 (1H, s).

### Example 7-1

### Preparation of N-{[1-phenyl-4-(3-methoxyphenyl)piperidin-4-yl]methyl}-N'-(4-amino-2,6-diisopropylphenyl)urea

To a solution of N-{[1-phenyl-4-(3-methoxyphenyl)piperidin-4-yl]methyl}-N'-(4-tert-butoxycarbonylamino-2,6-diisopropylphenyl)urea (188 mg, 0.306 mmol) in methanol (4 ml) is added 10 % hydrochloric acid/methanol (0.40 ml), and the mixture is stirred at room temperature overnight. The solvent is evaporated under reduced pressure, and the residue is crystallized from hexane/ether to give the hydrochloride of the title compound (155 mg) as white crystals.
¹H-NMR δ (DMSO-d₆): 1.05 (12H, d, J = 6.78), 2.28-2.32 (2H, brdm), 2.49-2.58 (2H, brdm), 2.98-3.07 (2H, m), 3.36-3.79 (9H, brdm), 6.17 (1H, brd), 6.78-7.11 (5H, m), 7.30-7.53 (3H, m), 7.73-7.83 (3H, m).

### Example 7-2

### Preparation of N-{[1-(2-methoxyphenyl)-4-(3-hydroxyphenyl)piperidin-4-yl]methyl}-N'-(4-amino-2,6-diisopropylphenyl)urea

The hydrochloride of the title compound is prepared in a similar manner to Example 7-1.

Melting point: 238-240°C

### Example 7-3

### Preparation of N-{[1-(2-benzyloxyphenyl)-4-(3-methoxyphenyl)piperidin-4-yl]methyl}-N'-(4-amino-2,6-diisopropylphenyl)urea

The hydrochloride of the title compound is prepared in a similar manner to Example 7-1.

Melting point: 201-202°C

### Example 7-4

### Preparation of N-{[1-(2-hydroxyphenyl)-4-(3-methoxyphenyl)piperidin-4-yl]methyl}-N'-(4-amino-2,6-diisopropylphenyl)urea

The title compound is prepared in a similar manner to Example 7-1.

Melting point: 207-208°C

### Example 8

### Preparation of N-{[1-phenyl-4-(3-hydroxyphenyl)piperidin-4-yl]methyl}-N'-(4-amino-2,6-diisopropylphenyl)urea

To a solution of N-{[1-phenyl-4-(3-methoxyphenyl)piperidin-4-yl]methyl}-N'-(4-tert-butoxycarbonylamino-2,6-diisopropylphenyl)urea (309 mg, 0.503 mmol) in methylene chloride (10 ml) is added boron tribromide (0.143 ml, 1.51 mmol) under ice-cooling, and the mixture is stirred for 3 hours at room temperature. The reaction is quenched by addition of methanol, and the reaction solution is neutralized by addition of aqueous sodium hydrogen carbonate solution. The mixture is extracted twice with ethyl acetate, and the organic layer is washed twice with water, and dried over anhydrous magnesium sulfate. The solvent is evaporated under reduced pressure and crystallized from hexane/ether to give the title compound (180 mg) as white crystals.
¹H-NMR δ (DMSO-d₆): 1.02 (12H, brd), 1.90-2.00 (4H, brd), 2.87-2.40 (8H, m), 4.90 (2H, brd), 5.61 (1H, brd), 6.28 (2H, s), 6.61-7.18 (10H, brdm), 9.23 (1H, s).

### Example 9

### Preparation of N-({1-phenyl-4-[3-(3-hydroxypropoxy)phenyl]piperidin-4-yl}methyl)-N'-(4-amino-2,6-diisopropylphenyl)urea

A suspension of N-{[1-phenyl-4-(3-hydroxyphenyl)piperidin-4-yl]methyl}-N'-(4-tert-butoxycarbonylamino-2,6-diisopropylphenyl)urea (46.5 mg, 0.078 mmol) and potassium carbonate (31.3 mg, 0.227 mmol) in dimethylformamide (DMF, 10 ml) is stirred at room temperature for 30 minutes, and thereto is added 3-bromopropylbenzyl ether (0.020 ml, 0.113 mmol). The mixture is warmed to 60°C, and stirred for 4 hours. Further, to the mixture is added 3-bromopropylbenzyl ether (0.013 ml, 0.078 mmol) at room temperature, and the mixture is warmed to 60°C, and stirred for 3 hours. Water is added to the reaction mixture, and the mixture is extracted once with ethyl acetate. The organic layer is washed twice with water, dried over anhydrous magnesium sulfate, and the solvent is evaporated under reduced pressure. The reaction mixture is dissolved in 10 % hydrochloric acid/methanol (20 ml), and thereto is added a 50 % (w/w) palladium on carbon (10 mg). The mixture is stirred under hydrogen atmosphere for 2 days, and the mixture is rendered faintly alkaline with a saturated aqueous sodium hydrogen carbonate solution, and the mixture is extracted with ethyl acetate. The organic layer is washed twice with a saturated aqueous sodium hydrogen carbonate solution, dried over anhydrous magnesium sulfate, and the solvent is evaporated under reduced pressure. The residue is purified by preparative TLC to give the title compound (6.5 mg, 15 %) as amorphous.
High performance liquid chromatography/Mass spectroscopy
m/z 559.4 (M+H)
Retention time: 2.84 min.

### Example 10

### Preparation of N-[(1-phenyl-4-{3-[3-(1,3-dioxo-1,3-dihydro-2H-isoindol-2-yl)propoxy]phenyl}piperidin-4-yl)methyl]-N'-(4-tert-butoxycarbonylamino-2,6-diisopropylphenyl)urea

A suspension of N-{[1-phenyl-4-(3-hydroxyphenyl)piperidin-4-yl]methyl}-N'-(4-tert-butoxycarbonylamino-2,6-diisopropylphenyl)urea (1.82 g, 2.96 mmol) and potassium carbonate (1.23 g, 8.88 mmol) in dimethylformamide (DMF, 100 ml) is stirred at room temperature for 30 minutes, and thereto is added N-(3-bromopropyl)phthalimide (1.19 g, 4.44 mmol). The mixture is warmed to 60°C, and stirred for 4 hours. To the mixture is added additional N-(3-bromopropyl)phthalimide (793 mg, 2.96 mmol) at room temperature, and the mixture is warmed to 70°C, and stirred for 4 hours. To the mixture is added water, and the mixture is extracted with ethyl acetate. The organic layer was washed twice with water, dried over anhydrous magnesium sulfate. The solvent is evaporated under reduced pressure and the residue is purified by silica gel chromatography to give the title compound (2.76 g) as amorphous.
¹H-NMR δ (DMSO-d₆): 1.03 (12H, d, J = 6.39), 1.45 (9H, s), 1.87-2.10 (6H, m), 2.94-3.03 (4H, m), 3.30-3.32 (2H, brd), 3.77 (2H, t, J = 6.60), 5.67 (1H, brd), 6.68-6.73 (3H, m), 6.87-6.97 (3H, m), 7.13-7.25 (6H, m), 7.79-7.86 (4H, m), 9.09 (1H, s).

### Example 11-1

### Preparation of N-{(1-phenyl-4-{3-[3-(2,2,2-trifluoroethyl)carbonylaminopropoxy]phenyl}piperidin-4-yl)methyl}-N'-(4-amino-2,6-diisopropylphenyl)urea

N-[(1-Phenyl-4-{3-[3-(1,3-dioxo-1,3-dihydro-2H-isoindol-2-yl)-propoxy]phenyl}piperidin-4-yl)methyl]-N'-(4-tert-butoxycarbonylamino-2,6-diisopropylphenyl)urea (2.76 g, 3.50 mmol) is dissolved in a 30 % methylamine/ethanol (50 ml), and the mixture is stirred at room temperature overnight. The solvent is evaporated under reduced pressure, and the reaction mixture (250 mg, 0.380 mmol) is separated and dissolved in dimethylformamide (DMF, 10 ml). A solution of 3,3,3-trifluoropropionic acid (34.6 mg, 0.270 mmol), 1-ethyl-3-(3'-dimethylaminopropyl )carbodiimide hydrochloride (WSC, 31.0 mg, 0.162 mmol), 1-hydroxybenzotriazole hydrate (HOBt, 22.0 mg, 0.162 mmol), triethylamine (0.045 ml, 0.324 mmol) in dimethylformamide (DMF, 10 ml) is stirred at room temperature overnight. Water is added to the mixture, and the mixture is extracted with ethyl acetate. The organic layer is washed twice with water, and washed once with a saturated aqueous sodium hydrogen carbonate solution. The mixture is dried over anhydrous magnesium sulfate, and the solvent is evaporated under reduced pressure. The residue is purified by preparative TLC to give the purified product (179 mg, 61 %) as amorphous, which is dissolved in 10 % hydrochloric acid/methanol. The mixture is stirred overnight, and concentrated, and the residue is crystallized from chloroform/ ethyl acetate to give the hydrochloride of the title compound (143 mg) as white crystals.

Melting point: 197-198°C

### Example 11-2

### Preparation of N-({1-phenyl-4-[3-(3-cyclopropylcarbonylaminopropoxy)-phenyl]piperidin-4-yl}methyl)-N'-(4-amino-2,6-diisopropylphenyl)urea

The hydrochloride of the title compound is prepared in a similar manner to Example 11-1.

Melting point: 197-199°C

### Example 12-1

### Preparation of N-{[1-(3-methoxyphenyl)-4-(3-methoxyphenyl)piperidin-4-yl]methyl}-N'-(4-amino-2,6-diisopropylphenyl)urea

### (a) Preparation of N-{[1-(3-methoxyphenyl)-4-(3-methoxyphenyl)-piperidin-4-yl]methyl}-N'-(4-tert-butoxycarbonylamino-2,6-diisopropylphenyl)urea

To a solution of 4-hydroxy-2-(2-hydroxyethyl)-2-(3-methoxyphenyl)butanenitrile (70 mg, 0.195 mmol) in acetonitrile (6 ml) are added successively trifluoromethanesulfonic anhydride (0.11 ml, 0.626 mmol) and triethylamine (0.080 ml, 0.626 mmol) at a temperature of from -30°C to -20°C, and the mixture is stirred for 15 minutes. To the mixture are added m-anisidine (0.043 ml, 0.387 mmol) and triethylamine (0.066 ml, 0.387 mmol) at a temperature of from -30°C to -20°C, and the mixture is slowly warmed to room temperature, and stirred for 2 hours. The reaction is quenched by addition of water, and the mixture is extracted with ether. The organic layer is washed twice with water, dried over anhydrous magnesium sulfate, and the solvent is evaporated under reduced pressure. To a suspension of lithium aluminum hydride (24 mg, 0.639 mmol) in tetrahydrofuran (THF, 5 ml) is added conc. sulfuric acid (0.018 ml, 0.639 mmol) under ice-cooling, and the mixture is warmed to room temperature, and stirred for 30 minutes. A solution of the resultant in THF (1 ml) is stirred at room temperature for 3 hours, and aqueous ammonia is slowly added to the reaction mixture at room temperature to terminate the reaction. The reaction mixture is filtered on Celite, and the filtrate is concentrated under reduced pressure. The residue is dissolved in tetrahydrofuran (THF, 6 ml), and thereto is added tert-butyl 3,5-diisopropyl-4-({[(4-nitrophenyl)oxy]carbonyl}amino)phenylcabamate (93 mg, 0.204 mmol) at room temperature, and the mixture is stirred overnight. The reaction is quenched by addition of water, and the mixture is extracted with ethyl acetate. The organic layer is washed thrice with 0.1N aqueous sodium hydroxide solution, dried over magnesium sulfate, and the solvent is evaporated under reduced pressure. The residue is purified by preparative TLC to give the title compound (50.1 mg) as amorphous.
High performance liquid chromatography/Mass spectroscopy
m/z 645.4 (M+H)
Retention time: 3.99 min.

### (b) Preparation of N-{[1-(3-methoxyphenyl)-4-(3-methoxyphenyl)-piperidin-4-yl]methyl}-N'-(4-amino-2,6-diisopropylphenyl)urea

N-{[1-(3-Methoxyphenyl)-4-(3-methoxyphenyl)piperidin-4-yl]-methyl}-N'-(4-tert-butoxycarbonylamino-2,6-diisopropylphenyl)urea (50.6 mg, 0.0785 mmol) is dissolved in 10 % hydrochloric acid/methanol (10 ml), and the mixture is stirred overnight. The mixture is concentrated, and crystallized from ethyl acetate to give the hydrochloride of the title compound (31.8 mg) as white crystals.
Melting point: 194-195°C

The following compounds are prepared in a similar manner to Example 12-1.

### Example 12-2

### Preparation of N-{[1-(3-methylphenyl)-4-(3-methoxyphenyl)piperidin-4-yl]methyl}-N'-(4-amino-2,6-diisopropylphenyl)urea

Melting point: 204-205°C

### Example 12-3

### Preparation of N-{[1-(3-fluorophenyl)-4-(3-methoxyphenyl)piperidin-4-yl]methyl}-N'-(4-amino-2,6-diisopropylphenyl)urea

Melting point: 182-183°C

### Example 12-4

### Preparation of N-{[1-(3-trifluoromethylphenyl)-4-(3-methoxyphenyl)-piperidin-4-yl]methyl}-N'-(4-amino-2,6-diisopropylphenyl)urea

Melting point: 184-185°C

### Example 12-5

### Preparation of N-{[1-(2,5-dimethoxyphenyl)-4-(3-methoxyphenyl)-piperidin-4-yl]methyl}-N'-(4-amino-2,6-diisopropylphenyl)urea

Melting point: 210-212°C

### Example 12-6

### Preparation of N-{[1-(4-fluorophenyl)-4-(3-methoxyphenyl)piperidin-4-yl]methyl}-N'-(4-amino-2,6-diisopropylphenyl)urea

Melting point: 200-201°C

### Example 12-7

### Preparation of N-{[1-(2-methoxyphenyl)-4-(3-methoxyphenyl)piperidin-4-yl]methyl}-N'-(3-amino-2,6-diisopropylphenyl)urea

Melting point: 200-201°C

### Example 12-8

### Preparation of N-{[1-(2-methoxyphenyl)-4-(3-trifluoromethylphenyl)-piperidin-4-yl]methyl}-N'-(4-amino-2,6-diisopropylphenyl)urea

Melting point: 199-200°C

### Example 12-9

### Preparation of N-{[1-(2-methoxyphenyl)-4-phenylpiperidin-4-yl]methyl}-N'-(4-amino-2,6-diisopropylphenyl)urea

Melting point: 209-210°C

### Example 12-10

### Preparation of N-{[1-(2-methoxyphenyl)-4-(3-fluorophenyl)piperidin-4-yl]methyl}-N'-(4-amino-2,6-diisopropylphenyl)urea

Melting point: 214-215°C

### Example 12-11

### Preparation of N-{[1-(2-methoxyphenyl)-4-(3-benzyloxyphenyl)piperidin-4-yl]methyl}-N'-(4-amino-2,6-diisopropylphenyl)urea

Melting point: 198-200°C

### Example 12-12

### Preparation of N-{[1-(2-methoxyphenyl)-4-(2-methoxyphenyl)piperidin-4-yl]methyl}-N'-(4-amino-2,6-diisopropylphenyl)urea

Melting point: 114-116°C

### Example 12-13

### Preparation of N-{[1-(2-methoxyphenyl)-4-(4-methoxyphenyl)piperidin-4-yl]methyl}-N'-(4-amino-2,6-diisopropylphenyl)urea

Melting point: 207-209

### Example 12-14

### Preparation of N-{[1-(5-chloro-2-methoxyphenyl)-4-(3-methoxyphenyl)-piperidin-4-yl]methyl}-N'-(4-amino-2,6-diisopropylphenyl)urea

Melting point: 202-203°C

### Example 12-15

### Preparation of N-{[1-(5-methyl-2-methoxyphenyl)-4-(3-methoxyphenyl)-piperidin-4-yl]methyl}-N'-(4-amino-2,6-diisopropylphenyl)urea

Melting point: 216-217°C

### Example 13

### Preparation of N-{[1-(3-pyridyl)-4-(3-methoxyphenyl)piperidin-4-yl]-methyl}-N'-(4-amino-2,6-diisopropylphenyl)urea

The hydrochloride of the title compound is prepared in a similar manner to Reference Example 2-2, Example 6 and Example 7.

Melting point: 184-185°C

### Example 14

The following compounds are prepared in a similar manner to Reference Example 7 and Example 13.

### Example 14-1

### Preparation of N-{[1-(4-pyridyl)-4-(3-methoxyphenyl)piperidin-4-yl]-methyl}-N'-(4-amino-2,6-diisopropylphenyl)urea

Melting point: 197-198°C

### Example 14-2

### Preparation of N-{[1-(2-pyridyl)-4-(3-methoxyphenyl)piperidin-4-yl]-methyl}-N'-(4-amino-2,6-diisopropylphenyl)urea

Melting point: 197-198°C

### Example 15-1

### Preparation of N-{[1-(2-methoxyphenyl)-4-(3-hydroxyphenyl)piperidin-4-yl]methyl}-N'-(4-tert-butoxycarbonylamino-2,6-diisopropylphenyl)urea

A suspension of N-{[1-(2-methoxyphenyl)-4-(3-benzyloxyphenyl)-piperidin-4-yl]methyl}-N'-(4-tert-butoxycarbonylamino-2,6-diisopropylphenyl)urea (405 mg, 0.562 mmol) and palladium on carbon (40 mg) in methanol (20 ml) is stirred at room temperature under hydrogen atmosphere for 6 hours. The mixture is filtered on Celite, and the solvent is evaporated under reduced pressure. The residue is purified by silica gel chromatography to give the title compound (293 mg) as amorphous.
High performance liquid chromatography/Mass spectroscopy
m/z 631.7 (M+H)
Retention time: 3.12 min.

### Example 15-2

### Preparation of N-{[1-(2-hydroxyphenyl)-4-(3-methoxyphenyl)piperidin-4-yl]methyl}-N'-(4-tert-butoxycarbonylamino-2,6-diisopropylphenyl)urea

The title compound is prepared in a similar manner to Example 15-1.
High performance liquid chromatography/Mass spectroscopy
m/z 721.3 (M+H)
Retention time: 4.15 min.

### Example 15-3

### Preparation of tert-butyl (4-{[({[1-(3-hydroxypyridin-2-yl)-4-(3-methoxyphenyl)piperidin-4-yl]methyl}amino)carbonyl]amino}-3,5-diisopropyl)-carbamate

The title compound is obtained in a similar manner to Example 15-1.
High performance liquid chromatography/ Mass spectroscopy
m/z 632.5 (M+H)
Retention time: 3.15 min.

### Example 16-1

### Preparation of N-{[1-(2-methoxyphenyl)-4-(3-butoxyphenyl)piperidin-4-yl]methyl}-N'-(4-amino-2,6-diisopropylphenyl)urea

To a solution of N-{[1-(2-methoxyphenyl)-4-(3-hydroxyphenyl)-piperidin-4-yl]methyl}-N'-(4-tert-butoxycarbonylamino-2,6-diisopropylphenyl)urea (50 mg, 0.0793 mmol) and cesium carbonate (32.9 mg, 0.238 mmol) in dimethylformamide (DMF, 10 ml) is added 1-iodobutane (0.014 ml, 0.119 mmol) at room temperature, and the mixture is warmed to 50°C, and stirred for 3 hours. Further, to the mixture are added potassium carbonate (87.0 mg, 0.63 mmol) and 1-iodobutane (0.028 ml, 0.238 mmol), and the mixture is stirred for 3 hours. The reaction is quenched by addition of water, and the mixture is extracted with ethyl acetate. The organic layer is washed twice with water, dried over anhydrous magnesium sulfate, and the solvent is evaporated under reduced pressure. The reaction mixture is dissolved in a 4N hydrochloric acid/dioxane (5 ml), and the mixture is stirred at room temperature overnight. The solvent is evaporated under reduced pressure, and the resultant is crystallized from ethyl acetate to give the hydrochloride of the title compound (35.2 mg) as white crystals.

Melting point: 192-193°C

### Example 16-2

### N-{[1-(2-Butoxyphenyl)-4-(3-methoxyphenyl)piperidin-4-yl]methyl}-N'-(4-amino-2,6-diisopropylphenyl)urea

The hydrochloride of the title compound is prepared in a similar manner to Example 16-1.

Melting point: 193-194°C

### Example 16-3

### N-(4-Amino-2,6-diisopropylphenyl)-N'-{[1-(3-methoxypyridin-2-yl)-4-(3-methoxyphenyl)piperidin-4-yl]methyl}urea

The hydrochloride of the title compound is prepared in a similar manner to Example 16-1.

Melting point: 188-189°C

### Example 16-4

### N-(4-Amino-2,6-diisopropylphenyl)-N'-{[1-[(3-propoxypyridin-2-yl)-4-(3-methoxyphenyl)piperidin-4-yl]methyl]urea

The hydrochloride of the title compound is prepared in a similar manner to Example 16-1.

Melting point: 186-188°C

### Example 16-5

### N-(4-Amino-2,6-diisopropylphenyl)-N'-{[1-[(3-isopropoxypyridin-2-yl)-4-(3-methoxyphenyl)piperidin-4-yl]methyl]urea

The hydrochloride of the title compound is prepared in a similar manner to Example 16-1.

Melting point: 190-191°C

### Example 16-6

### N-(4-Amino-2,6-diisopropylphenyl)-N'-{[1-[3-(2,2,2-trifluoroethoxy)-pyridin-2-yl]piperidin-4-yl]methyl}urea

The hydrochloride of the title compound is prepared in a similar manner to Example 16-1.

Melting point: 189-191°C

### Example 16-7

### N-(4-Amino-2,6-diisopropylphenyl)-N'-{[1-[3-(2-methoxyethoxy)pyridin-2-yl]-4-(3-methoxyphenyl)piperidin-4-yl]methyl}urea

The hydrochloride of the title compound is prepared in a similar manner to Example 16-1.

Melting point: 196-197°C

### Example 16-8

### N-(4-Amino-2,6-diisopropylphenyl)-N'-{[1-[3-(2-ethoxyethoxy)pyridin-2-yl]-4-(3-methoxyphenyl)piperidin-4-yl]methyl}urea

The hydrochloride of the title compound is prepared in a similar manner to Example 16-1.

Melting point: 197-198°C

### Example 16-9

### N-(4-Amino-2,6-diisopropylphenyl)-N'-{[1-[3-(2-phenoxyethoxy)pyridin-2-yl]-4-(3-methoxyphenyl)piperidin-4-yl]methyl}urea

The hydrochloride of the title compound is prepared in a similar manner to Example 16-1.

Melting point: 190-191°C

### Example 16-10

### N-(4-Amino-2,6-diisopropylphenyl)-N'-({4-(3-methoxyphenyl)-1-[3-(3-methoxypropoxy)pyridin-2-yl]piperidin-4-yl}methyl)urea

The hydrochloride of the title compound is prepared in a similar manner to Example 16-1.

Melting point: 174-175°C

### Example 17

### Preparation of N-{[1-(2-benzyloxyphenyl)-4-(3-methoxyphenyl)piperidin-4-yl]methyl}-N'-(4-tert-butoxycarbonylamino-2,6-diisopropylphenyl)urea

To a suspension of lithium aluminum hydride (52.0 mg, 1.39 mmol) in ether (20 ml) is added a solution of 1-(2-benzyloxyphenyl)-4-(3-methoxyphenyl)piperidin-4-carbonitrile (184 mg, 0.462 mmol) in ether (15 ml) at room temperature, and the mixture is heated under reflux for 3 hours. To the mixture is added slowly aqueous ammonia at room temperature, and the reaction is quenched. The mixture is filtered on Celite, and the reaction solution is evaporated under reduced pressure. The residue is dissolved in tetrahydrofuran (THF, 10 ml), and thereto is added tert-butyl 3,5-diisopropyl-4-({[(4-nitrophenyl)oxy]-carbonyl}amino)phenylcarbamate (196 mg, 0.426 mmol) at room temperature. The mixture is stirred for 3 hours, and the reaction is quenched by additon of a saturated aqueous potassium carbonate. The mixture is extracted with ethyl acetate, and the organic layer is washed four times with a saturated aqueous potassium carbonate solution. The mixture is dried over anhydrous magnesium sulfate, and the resultant is evaporated under reduced pressure. The residue is purified by silica gel chromatography to give the title compound (246 mg) as amorphous.
¹H-NMR δ (DMSO-d₆): 1.02 (12H, d, J = 6.60), 1.98-2.07 (4H, m), 2.94-2.99 (4H, m), 3.16 (2H, brdm), 3.76 (3H, s), 5.09 (2H, s), 5.63 (1H, brd.), 6.84-7.45 (17H, m), 9.09 (1H, brd.).

### Example 18-1

### Preparation of N-({1-[2-(3-hydroxypropoxy)phenyl]-4-(3-methoxyphenyl)-piperidin-4-yl}methyl)-N'-(4-amino-2,6-diisopropylphenyl)urea

To a solution of N-{[1-(2-hydroxyphenyl)-4-(3-methoxyphenyl)-piperidin-4-yl]methyl}-N'-(4-tert-butoxycarbonylamino-2,6-diisopropylphenyl)urea (30 mg, 0.0476 mmol) and potassium carbonate (33 mg, 0.238 mmol) in dimethylformamide (DMF, 10 ml) is added 3-bromopropyl acetate (34 mg, 0.190 mmol) at room temperature, and the mixture is warmed to 55°C, and stirred for 5 hours. The reaction is quenched by addition of water, and the mixture is extracted with ethyl acetate. The organic layer is washed twice with water, dried over anhydrous magnesium sulfate, and the solvent is evaporated under reduced pressure. The reaction mixture is dissolved in methanol (5 ml)/water (1 ml), and thereto is added potassium carbonate (80 mg, 0.579 mmol) at room temperature. The mixture is stirred for 3 hours, and the reaction is quenched by addition of water. The mixture is extracted with ethyl acetate, and the organic layer is washed twice with a saturated aqueous ammonium chloride solution, and washed once with a saturated aqueous sodium hydrogen carbonate solution. The resultant is dried over anhydrous magnesium sulfate, and the solvent is evaporated under reduced pressure. The reaction mixture is dissolved in 4N hydrochloric acid/dioxane (5 ml), and the mixture is stirred overnight. The solvent is evaporated under reduced pressure, and the residue is crystallized from ethyl acetate to give the hydrochloride of the title compound (14.3 mg) as white crystals.

Melting point: 188-189°C

### Example 18-2

### Preparation of N-({1-(2-methoxyphenyl)-4-[3-(3-hydroxypropoxy)phenyl]-piperidin-4-yl}methyl)-N'-(4-amino-2,6-diisopropylphenyl)urea

The title compound is prepared in a similar manner to Example 18-1.

Melting point: 203-205°C

### Example 18-3

### N-(4-Amino-2,6-diisopropylphenyl)-N'-{[1-[3-(2-hydroxyethoxy)pyridin-2-yl]-4-(3-methoxyphenyl)piperidin-4-yl]methyl}urea

The hydrochloride of the title compound is prepared in a similar manner to Example 18-1.

Melting point: 174-176°C

### Example 19

### tert-Butyl (4-{[({[1-[3-(benzyloxy)pyridin-2-yl]-4-(3-methoxyphenyl)-piperidin-4-yl]methyl}amino)carbonyl]amino}-3,5-diisopropylphenyl)-carbamate

The title compound is prepared in a similar manner to Reference Example 2 and Example 6-1.
High performance liquid chromatography/Mass spectroscopy
m/z 722.4 (M+H)
Retention time: 3.57 min.

### Example 20-1

### N-(4-Amino-2,6-diisopropylphenyl)-N'-{[1-(3-hydroxypyridin-2-yl)-4-(3-methoxyphenyl)piperidin-4-yl]methyl}urea

tert-Butyl (4-{[({[1-(3-hydroxypyridin-2-yl)-4-(3-methoxyphenyl)-piperidin-4-yl]methyl}amino)carbonyl]amino}-3,5-diisopropylphenyl)-carbamate (6.80 mg, 0.0108 mmol) is dissolved in a 4N solution of hydrochloric acid in dioxane (5 ml), and the mixture is stirred overnight. The reaction solution is concentrated, and crystallized from ethyl acetate to give the hydrochloride of the title compound (5.60 mg) as white crystals.

Melting point: 199-201°C

### Example 20-2

### N-(4-Amino-2,6-diisopropylphenyl)-N'-{[1-[(3-benzyloxy)pyridin-2-yl]-4-(3-methoxyphenyl)piperidin-4-yl]methyl}urea

The hydrochloride of the title compound is prepared in a similar manner to Example 20-1.

Melting point: 171-172°C

In the above Examples and Reference Examples, the high performance liquid chromatography for retention time is carried out under the following conditions.
Column:
Octadecyl-chemical bond-type silica (ODS), Pore size: 5 µm, Particle size: 12 nm, Column length: 50 mm, Column inner diameter: 4.6 mm (trade name: YMC CombiScreen ODS-A (S-5 µm, 12 nm) 50x4.6 mm (YMC Co., Ltd.))
Flow rate: 3.5 ml/min.
Wavelength for detection: 220 nm
Mobile phase:
Solution A: aqueous 0.05 % trifluoroacetic acid solution
Solution B: 0.035 % solution of trifluoroacetic acid in acetonitrile
Time program:

| Step | Time (min) | Solution A:Solution B |
|---|---|---|
| 1 | 0.0 - 0.5 | 90:10 |
| 2 | 0.5-4.2 | 90:10 → 1:99 |
| 3 | 4.2-4.4 | 1:99 → 99:1 |

As the compound of the present invention represented by the above formula (1), the following compounds are also exemplified in addition to the compounds as disclosed in Examples.
N-(4-amino-2,6-diisopropylphenyl)-N'-{[1-[2-(2-methoxyethoxy)-phenyl]-2-(3-methoxyphenyl)piperidin-2-yl]methyl}urea
N-(4-amino-2,6-diisopropylphenyl)-N'-{[1-[2-(2-methoxyethoxy)-phenyl]-3-(3-methoxyphenyl)piperidin-3-yl]methyl}urea
N-{[(4-amino-2,6-diisopropylphenyl)amino]carbonyl}-1-[2-(2-methoxyethoxy)phenyl]-4-(3-methoxyphenyl)piperidin-4-carboxamide
N-(4-amino-2,6-diisopropylphenyl)-N'-{[1-[2-(2-methoxyethoxy)-phenyl]-4-(3-methoxyphenyl)-2,6-dimethylpiperidin-4-yl]methyl}urea
N-(4-amino-2,6-diisopropylphenyl)-N'-{[1-[2-(2-methoxyethoxy)-phenyl]-4-(3-methoxyphenyl)-2,6-dioxopiperidin-4-yl]methyl}urea

### EXPERIMENTS

The ACAT inhibitory activity of the present compounds is evaluated by the following method.

### 1. Assay of ACAT inhibitory activity using Caco-2 cells derived from human colon adenocarcinoma:

The measurement of ACAT inhibitory activity using Caco-2 cells was carried out by a modified method of that disclosed in the literature (J. Lipid. Research, 28, 1057-1066, 1987, Jpn. J. Pharmacol., 68, 191-199, 1995), using radioactive [1-³H]oleic acid as a substrate, and the enzymatic activity was calculated from the radioactivity of the resultant labeled cholesterolyl oleate. The results are shown in Table 1.

**Table 1**

| Test compound (Example No.) | ACAT inhibitory rate (%) Concentration of compound: 500 nM |
|---|---|
| 11-1 | 57 |
| 12-2 | 59 |
| 12-3 | 63 |

### 2. Assay of ACAT inhibitory activity in a macrophage derived from rat peritoneal cavity:

The rat peritoneal-derived macrophage was prepared according to the method disclosed in the literature: Biochimica et Biophysica Acta, 1126, 73-80, 1992. The ACAT activity was determined by a modified method of the method disclosed in the above literature: Biochimica et Biophysica Acta, 1126, 73-80, 1992, using radioactive [9,10-³H]oleic acid and exogenous cholesterol contained in the liposome which was reconstituted according to the method disclosed in the literature: Biochimica et Biophysica Acta, 1213, 127-134, 1994, and calculated from the radioactivity of the labeled cholesterolyl oleate. The results are shown in Table 2.

**Table 2**

| Test compound (Example No.) | ACAT inhibitory rate (%) Concentration of compound: 500 nM |
|---|---|
| 11-1 | 99 |
| 12-2 | 99 |
| 12-3 | 99 |

### INDUSTRIAL APPLICABILITY

The piperidine derivative of the present invention or an acid addition salt thereof strongly inhibits ACAT activity in Caco-2 cells derived from human colon adenocarcinoma and in rat peritoneal-derived macrophage. Therefore, the present piperidine derivative is useful not only as an agent for treatment of hyperlipidemia, but also in the prophylaxis or treatment of atherosclerosis per se or various diseases accompanied by atherosclerosis, for example, cerebral infarction, cerebral thrombosis, transient cerebral ischemia, angina pectoris, myocardial infarction, peripheral thrombus or occlusion.

## Claims

1. A compound of the formula (1): 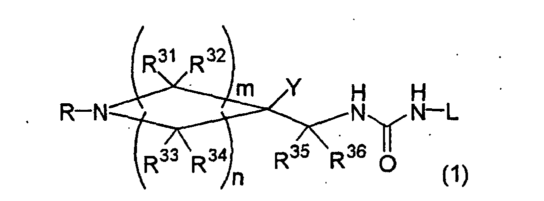 wherein m and n are independently an integer of 0 to 4, and m+n=4,
L is a cycloalkyl group, a substituted cycloalkyl group, an aromatic group, or a substituted aromatic group,
Y is an aryl group or a substituted aryl group,
R is a hydrogen atom, an alkyl group, a substituted alkyl group, an alkenyl group, a substituted alkenyl group, an alkynyl group, a substituted alkynyl group, a cycloalkyl group, a substituted cycloalkyl group, an aromatic group, a substituted aromatic group, or a group of the formula: -C(=O)R² (R² is an alkyl group, a substituted alkyl group, an alkenyl group, a substituted alkenyl group, an alkynyl group, a substituted alkynyl group, a cycloalkyl group, a substituted cycloalkyl group, an aromatic group, or a substituted aromatic group),
R³¹, R³², R³³ and R³⁴ are the same or different, and are selected, if two or more thereof exist, independently from a hydrogen atom, an alkyl group, a substituted alkyl group, a hydroxy group, an alkoxy group, and an aralkyloxy group, or a combination of R³¹ and R³², and/or a combination of R³³ and R³⁴ may combine each other and form an oxo group,
R³⁵ and R³⁶ are the same or different, and are selected, if both exist, independently from a hydrogen atom, an alkyl group and a substituted alkyl group, or R³⁵ and R³⁶ may combined each other and form an oxo group,
or a prodrug thereof, or a pharmaceutically acceptable salt of the same.

2. The compound according to claim 1, or a prodrug thereof, or a pharmaceutically acceptable salt of the same, wherein Y is a phenyl group or a substituted phenyl group.

3. The compound according to claim 2, or a prodrug thereof, or a pharmaceutically acceptable salt of the same, wherein L is a substituted phenyl group.

4. The compound according to claim 3, or a prodrug thereof, or a pharmaceutically acceptable salt of the same, wherein L is a group of the formula (2): 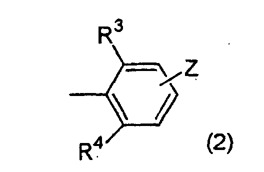 wherein R³ and R⁴ are independently a substituted or unsubstituted lower alkyl group,
Z is a hydrogen atom, a hydroxy group, a lower alkylsulfonamido group, a lower alkoxycarbonylamino group, an amino group, a lower alkylamino group, or a di-lower alkyl amino group.

5. The compound according to claim 4, or a prodrug thereof, or a pharmaceutically acceptable salt of the same, wherein R is an aromatic group or a substituted aromatic group.

6. The compound according to claim 5, or a prodrug thereof, or a pharmaceutically acceptable salt of the same, wherein R is a substituted phenyl group.

7. The compound according to claim 6, or a prodrug thereof, or a pharmaceutically acceptable salt of the same, wherein Y is a substituted phenyl group or a substituted pyridyl group, and said phenyl group or pyridyl group may be substituted by one or more groups, which are the same or different and selected from a hydroxy group and a group of the formula: -O-E-A {O is an oxygen atom, E is a divalent C₁₋₈ hydrocarbon group optionally having an unsaturated bond, and A is a hydrogen atom, a hydroxy group, a carboxyl group, a lower alkoxycarbonyl group, a benzyloxycarbonyl group, a halogen atom, a cyano group, a trifluoromethyl group, an aralkyloxy group, an aryloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, or a group of the formula:-NR⁶R⁷ (R⁶ and R⁷ are independently a hydrogen atom, a lower alkyl group, a lower alkoxy-substituted lower alkyl group, a cycloalkyl group, a lower alkoxycarbonyl group, a heteroarylmethyl group, or an aralkyl group, or R⁶ and R⁷ may combine each other, and with the adjacent nitrogen atom to which they bond, form a saturated cyclic amino group having 3 to 8 carbon atoms as ones forming the said ring, and optionally having one -NR⁸- (R⁸ is a hydrogen atom, a lower alkyl group, a phenyl group, a lower alkoxycarbonyl group, or a benzyl group) or one oxygen atom in the cycle thereof), a group of the formula: -C(=O)NR⁶R⁷ (R⁶ and R⁷ are as defined above), or a group of the formula:-NHC(=O)R⁹ (R⁹ is an alkyl group, a substituted alkyl group, a cycloalkyl group, or a substituted cycloalkyl group)}.

8. The compound according to claim 7, or a prodrug thereof, or a pharmaceutically acceptable salt of the same, wherein in at least one of the substituents represented by the formula: -O-E-A on Y, E is a C₁₋₄ alkylene group.

9. The compound according to claim 8, or a prodrug thereof, or a pharmaceutically acceptable salt of the same, wherein in at least one of the substituents represented by the formula: -O-E-A on Y, A is a hydrogen atom or a hydroxy group.

10. The compound according to claim 9, or a prodrug thereof, or a pharmaceutically acceptable salt of the same, wherein Z is a hydrogen atom or an amino group.

11. The compound according to claim 10, or a prodrug thereof, or a pharmaceutically acceptable salt of the same, wherein R³ and R⁴ are independently an unsubstituted lower alkyl group.

12. The compound according to claim 11, or a prodrug thereof, or a pharmaceutically acceptable salt of the same, wherein R is a substituted phenyl group or a substituted pyridyl group, and said phenyl group or pyridyl group may be substituted by one or more groups, which are the same or different and selected from a hydroxy group and a group of the formula: -O-E-A {O is an oxygen atom, E is a divalent C₁₋₈ hydrocarbon group optionally having an unsaturated bond, and A is a hydrogen atom, a hydroxy group, a carboxyl group, a lower alkoxycarbonyl group, a benzyloxycarbonyl group, a halogen atom, a cyano group, a trifluoromethyl group, an aralkyloxy group, an aryloxy group, a lower alkoxy group, a lower alkanoyloxy group, a lower alkylthio group, a lower alkylsulfinyl group, a lower alkylsulfonyl group, an alkyl-substituted or unsubstituted benzenesulfonyloxy group, a lower alkanoylamino group, a lower alkoxycarbonylamino group, a lower alkylsulfonamido group, a phthalimido group, a cycloalkyl group, an aryl group, a substituted aryl group, a heteroaryl group, a substituted heteroaryl group, or a group of the formula: -NR⁶R⁷ (R⁶ and R⁷ are independently a hydrogen atom, a lower alkyl group, a lower alkoxy-substituted lower alkyl group, a cycloalkyl group, a lower alkoxycarbonyl group, a heteroarylmethyl group, or an aralkyl group, or R⁶ and R⁷ may combine each other; and with the adjacent nitrogen atom to which they bond, form a saturated cyclic amino group having 3 to 8 carbon atoms as ones forming the said ring, and optionally having one -NR⁸- (R⁸ is a hydrogen atom, a lower alkyl group, a phenyl group, a lower alkoxycarbonyl group, or a benzyl group) or one oxygen atom in the cycle thereof), or a group of the formula: -C(=O)NR⁶R⁷ (R⁶ and R⁷ are as defined above)}.

13. The compound according to claim 12, or a prodrug thereof, or a pharmaceutically acceptable salt of the same, wherein in at least one of the substituents represented by the formula:-O-E-A on R, E is a C₁₋₄ alkylene group.

14. The compound according to claim 13, or a prodrug thereof, or a pharmaceutically acceptable salt of the same, wherein in at least one of the substituents represented by the formula:-O-E-A on R, A is a hydrogen atom, a hydroxy group, a lower alkoxy group, a lower alkanoyloxy group, a lower alkanoylamino group, a lower alkoxycarbonylamino group, or a group of the formula: -NR⁶R⁷ or the formula: -C(=O)NR⁶R⁷.

15. The compound according to claim 14, or a prodrug thereof, or a pharmaceutically acceptable salt of the same, wherein in at least one of the substituents represented by the formula:-O-E-A on R, A is a hydrogen atom, a hydroxy group, a lower alkoxy group, or a group of the formula: -NR⁶R⁷ (R⁶ and R⁷ are independently a hydrogen atom or a lower alkyl group).

16. The compound according to claim 15, or a prodrug thereof, or a pharmaceutically acceptable salt of the same, wherein R³ and R⁴ are an isopropyl group.

17. The compound according to any one of claims 1 to 16, or a prodrug thereof, or a pharmaceutically acceptable salt of the same, wherein m is 2, n is 2, and all of R³¹, R³², R³³, R³⁴, R³⁵ and R³⁶ are a hydrogen atom.

18. A pharmaceutical composition comprising as an active ingredient the compound as set forth in any one of claims 1 to 17, or a prodrug thereof, or a pharmaceutically acceptable salt of the same.

19. An acyl-CoA: cholesterol acyl transferase (ACAT) inhibitor, which comprises as an active ingredient the compound as set forth in any one of claims 1 to 17, or a prodrug thereof, or a pharmaceutically acceptable salt of the same.

20. An agent for treatment of hyperlipidemia or atherosclerosis, which comprises as an active ingredient the compound as set forth in any one of claims 1 to 17, or a prodrug thereof, or a pharmaceutically acceptable salt of the same.

21. A method for treatment of hyperlipidemia or atherosclerosis in a patient in need, which comprises administering a therapeutically effective amount of the compound as set forth in any one of claims 1 to 17, or a prodrug thereof, or a pharmaceutically acceptable salt of the same, to said patient.

22. A use of the compound as set forth in any one of claims 1 to 17, or a prodrug thereof, or a pharmaceutically acceptable salt of the same, in preparation of an agent for treatment of hyperlipidemia or atherosclerosis.
